# EUROPEAN PATENT APPLICATION

(11) **EP 1 953 151 A1**
(43) Date of publication of application: **06.08.2008**
(21) Application number: 06832860.8
(22) Date of filing: 17.11.2006
(51) Int. Cl.: C07D 401/10, A61P 25/28, A61K 31/454

(54) **SALTS OF CYNNAMIDE COMPOUND OR SOLVATES THEREOF**

(30) Priority: 18.11.2005 JP 2005333718
(71) Applicant: Eisai R&D Management Co., Ltd., Tokyo 112-8088 (JP)
(72) Inventor: KUSHIDA, Ikuo, Tsukuba-shi Ibaraki 300-2635 (JP); DOI, Eriko, Tsukuba-shi Ibaraki 300-2635 (JP); ITO, Koichi, Tsukuba-shi Ibaraki 300-2635 (JP); NAKAMURA, Taiju, Kamisu-shi Ibaraki 314-0255 (JP)
(74) Representative: HOFFMANN EITLE
(86) International application number: PCT/JP2006/322981
(87) International publication number: WO 2007/058304

(57) **Abstract**

The invention provides crystals of dihydrochloride monohydrate of the compound of the following formula having an Aβ production inhibiting effect which crystals are **characterized by** exhibiting a diffraction peak at an angle of diffraction (2θ ± 0.2°) of 10.9° in powder X-ray diffractometry. Further, the invention also provides the compound in the form of various salts, crystal forms and amorphous forms which are suitable for the development of drugs.

## Description

### TECHNICAL FIELD

The present invention relates to a salt of a cinnamide compound having an effect of reducing amyloid-β production, or a solvate thereof.

### BACKGROUND ART

Alzheimer's disease is a disease characterized by degeneration and loss of neurons as well as formation of senile plaques and neurofibrillary degeneration. Currently, Alzheimer's disease is treated only with symptomatic treatment using a symptom improving agent typified by an acetylcholinesterase inhibitor, and a fundamental remedy to inhibit progression of the disease has not yet been developed. It is necessary to develop a method for controlling the cause of the onset of pathology in order to create a fundamental remedy for Alzheimer's disease.
It is assumed that Aβ-proteins as metabolites of amyloid precursor proteins (hereinafter referred to as APP) are highly involved in degeneration and loss of neurons and onset of symptoms of dementia (see Non-Patent Documents 1 and 2, for example). An Aβ-protein has, as main components, Aβ40 consisting of 40 amino acids and Aβ42 with two amino acids added at the C-terminal. The Aβ40 and Aβ42 are known to have high aggregability (see Non-Patent Document 3, for example) and to be main components of senile plaques (see Non-Patent Documents 4 and 5, for example). Further, it is known that the Aβ40 and Aβ42 are increased by mutation in APP and presenilin genes which is observed in familial Alzheimer's disease (see Non-Patent Documents 6, 7 and 8, for example). Accordingly, a compound that reduces production of Aβ40 and Aβ42 is expected as a progression inhibitor or prophylactic agent for Alzheimer's disease.
Non-Patent Document 1: Klein WL, and seven others, Alzheimer's disease-affected brain: Presence of oligomeric Aβ ligands (ADDLs) suggests a molecular basis for reversible memory loss, Proceding National Academy of Science USA 2003, Sep 2; 100(18), p.10417-10422.
Non-Patent Document 2: Nitsch RM, and 16 others, Antibodies against β-amyloid slow cognitive decline in Alzheimer's disease, Neuron, 2003, May 22; 38, p.547-554.
Non-Patent Document 3: Jarrett JT, and two others, The carboxy terminus of the β amyloid protein is critical for the seeding of amyloid formation: Implications for the pathogenesis of Alzheimers' disease, Biochemistry, 1993, 32(18), p.4693-4697.
Non-Patent Document 4: Glenner GG, and one other, Alzheimer's disease: initial report of the purification and characterization of a novel cerebrovascular amyloid protein, Biochemical and biophysical research communications, 1984, May 16, 120(3), p.885-890.
Non-Patent Document 5: Masters CL, and five others, Amyloid plaque core protein in Alzheimer disease and Down syndrome, Proceding National Academy of Science USA, 1985, Jun, 82(12), p.4245-4249.
Non-Patent Document 6: Gouras GK, and 11 others, Intraneuronal Aβ42 accumulation in human brain, American Journal of Pathology, 2000, Jan, 156(1), p.15-20.
Non-Patent Document 7: Scheuner D, and 20 others, Secreted amyloid β-protein similar to that in the senile plaques of Alzheimer's disease is increased in vivo by the presenilin 1 and 2 and APP mutations linked to familial Alzheimer's disease, Nature Medicine, 1996, Aug, 2(8), p.864-870.
Non-Patent Document 8: Forman MS, and four others, Differential effects of the swedish mutant amyloid precursor protein on β-amyloid accumulation and secretion in neurons and nonneuronal cells, The Journal of Biological Chemistry, 1997, Dec 19, 272(51), p.32247-32253.

### DISCLOSURE OF THE INVENTION

Properties of compounds useful as pharmaceuticals and salts thereof and crystal forms and amorphous forms thereof greatly affect bioavailability of drugs, purity of drug substances, formulation of preparations and the like. Therefore, in development of pharmaceuticals, it is necessary to research which salts, crystal forms and amorphous forms of the compounds are the most excellent as pharmaceuticals. Specifically, since their properties depend on the attribution of the individual compounds, it is generally difficult to estimate salts, crystal forms and amorphous forms for drug substances having excellent properties and it is demanded to make various studies for each compound, actually.

The present inventors have found that (3E)-1-[(1S)-1-(4-fluorophenyl)ethyl]-3-[3-methoxy-4-(4-methyl-1H-imidazol-1-yl)benzylidene]piperidin-2-one represented by the following formula: 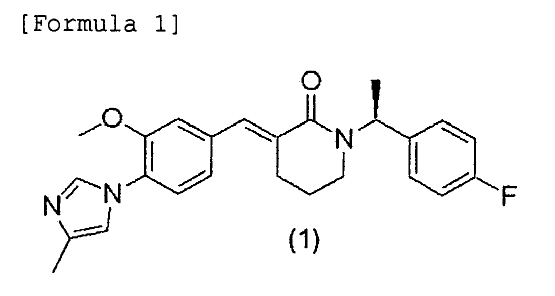 (hereinafter sometimes referred to as compound (1)) has an effect of reducing Aβ production and is effective for treatment of a neurodegenerative disease caused by Aβ such as Alzheimer's disease or Down's syndrome (PCT/JP2005/009537). The present inventors have isolated various salts, crystal forms and amorphous forms of the compound (1), understood their properties and morphology and made extensive studies. As a result, the inventors have found salts for drug substances having excellent properties, solvates thereof, crystals thereof, amorphous forms thereof and the like. This finding has led to the completion of the present invention.

Specifically, the present invention relates to:
[1]. A salt comprising one acid selected from the group consisting of an inorganic acid, an organic carboxylic acid and an organic sulfonic acid and (3E)-1-[(1S)-1-(4-fluorophenyl)ethyl]-3-[3-methoxy-4-(4-methyl-1H-imidazol-1-yl)benzylidene]piperidin-2-one, or a solvate thereof;
[2]. The salt or the solvate thereof according to [1] above, wherein the acid is an organic carboxylic acid;
[3]. The salt or the solvate thereof according to [1] or [2] above, wherein the organic carboxylic acid is acetic acid, oxalic acid, maleic acid, tartaric acid, malonic acid, fumaric acid or citric acid;
[4]. The salt or the solvate thereof according to [1] above, wherein the acid is an organic sulfonic acid;
[5]. The salt or the solvate thereof according to [1] or [4] above, wherein the organic sulfonic acid is methanesulfonic acid, trifluoromethanesulfonic acid, ethanesulfonic acid, benzenesulfonic acid, toluenesulfonic acid or camphorsulfonic acid;
[6]. The salt or the solvate thereof according to [1] above, wherein the acid is an inorganic acid;
[7]. The salt or the solvate thereof according to [1] or [6] above, wherein the inorganic acid is tetrafluoroboric acid, hydrofluoric acid, hydrochloric acid, hydrobromic acid, hydriodic acid, sulfuric acid, nitric acid, perchloric acid, phosphoric acid, carbonic acid or bicarbonic acid;
[8]. The salt or the solvate thereof according to [1], [6] or [7] above, wherein the inorganic acid is tetrafluoroboric acid, hydrofluoric acid, hydrochloric acid, hydrobromic acid or hydriodic acid;
[9]. The salt or the solvate thereof according to [1], [6], [7] or [8] above, wherein the inorganic acid is hydrochloric acid;
[10]. The salt or the solvate thereof according to [1], [6], [7] or [8] above, wherein the inorganic acid is hydrobromic acid;
[11]. The solvate according to any one of [1] to [10] above, wherein the solvate is a hydrate or an alcoholate;
[12]. The solvate according to [11] above, wherein the alcoholate is a 1-propanolate;
[13]. The salt or the solvate thereof according to any one of [1] to [12] above, which is a crystalline form or an amorphous form;
[14]. A crystal of (3E)-1-[(1S)-1-(4-fluorophenyl)ethyl]-3-[3-methoxy-4-(4-methyl-1H-imidazol-1-yl)benzylidene]piperidin-2-one dihydrochloride monohydrate having a diffraction peak at a diffraction angle (2θ ± 0.2°) of 10.9° in powder X-ray diffractometry;
[15]. The crystal according to [14] above, further having a diffraction peak at a diffraction angle (2θ ± 0.2°) of 11.1°;
[16]. The crystal according to [14] or [15] above, further having a diffraction peak at a diffraction angle (2θ ± 0.2°) of 12.7°;
[17]. The crystal according to any one of [14] to [16] above, further having a diffraction peak at a diffraction angle (2θ ± 0.2°) of 13.0°;
[18]. The crystal according to any one of [14] to [17] above, further having a diffraction peak at a diffraction angle (2θ ± 0.2°) of 17.2°;
[19]. The crystal according to any one of [14] to [18] above, further having a diffraction peak at a diffraction angle (2θ ± 0.2°) of 20.7°;
[20]. The crystal according to any one of [14] to [19] above, further having a diffraction peak at a diffraction angle (2θ ± 0.2°) of 24.5°;
[21]. The crystal according to any one of [14] to [20] above, further having a diffraction peak at a diffraction angle (2θ ± 0.2°) of 25.6°;
[22]. A crystal of (3E)-1-[(1S)-1-(4-fluorophenyl)ethyl]-3-[3-methoxy-4-(4-methyl-1H-imidazol-1-yl)benzylidene]piperidin-2-one dihydrobromide monohydrate having a diffraction peak at a diffraction angle (2θ ± 0.2°) of 10.5° in powder X-ray diffractometry;
[23]. The crystal according to [22] above, further having a diffraction peak at a diffraction angle (2θ ± 0.2°) of 13.2°;
[24]. The crystal according to [22] or [23] above, further having a diffraction peak at a diffraction angle (2θ ± 0.2°) of 17.7°;
[25]. The crystal according to [23] or [24] above, further having a diffraction peak at a diffraction angle (2θ ± 0.2°) of 21.4°;
[26]. The crystal according to any one of [23] to [25] above, further having a diffraction peak at a diffraction angle (2θ ± 0.2°) of 22.3°;
[27]. The crystal according to any one of [23] to [26] above, further having a diffraction peak at a diffraction angle (2θ ± 0.2°) of 39.4°;
[28]. A crystal of (3E)-1-[(1S)-1-(4-fluorophenyl)ethyl]-3-[3-methoxy-4-(4-methyl-1H-imidazol-1-yl)benzylidene]piperidin-2-one phosphate 1-propanolate having a diffraction peak at a diffraction angle (2θ ± 0.2°) of 8.4°;
[29]. The crystal according to [28] above, further having a diffraction peak at a diffraction angle (2θ ± 0.2°) of 14.3°;
[30]. The crystal according to [28] or [29] above, further having a diffraction peak at a diffraction angle (2θ ± 0.2°) of 19.3°;
[31]. The crystal according to any one of [28] to [30] above, further having a diffraction peak at a diffraction angle (2θ ± 0.2°) of 21.1°;
[32]. The crystal according to any one of [28] to [30] above, further having a diffraction peak at a diffraction angle (2θ ± 0.2°) of 29.6°;
[33]. (3E)-1-[(1S)-1-(4-fluorophenyl)ethyl]-3-[3-methoxy-4-(4-methyl-1H-imidazol-1-yl)benzylidene]piperidin-2-one phosphate which is an amorphous form;
[34]. (3E)-1-[(1S)-1-(4-fluorophenyl)ethyl]-3-[3-methoxy-4-(4-methyl-1H-imidazol-1-yl)benzylidene]piperidin-2-one monotartrate which is an amorphous form;
[35]. (3E)-1-[(1S)-1-(4-fluorophenyl)ethyl]-3-[3-methoxy-4-(4-methyl-1H-imidazol-1-yl)benzylidene]piperidin-2-one hemisulfate which is an amorphous form;
[36]. The amorphous compound according to any one of [33] to [35] above, not comprising a crystalline form; and
[37]. The amorphous form according to any one of [33] to [35] above, not having a diffraction peak in powder X-ray diffractometry.

The compound (1) of the present invention can be produced by the synthesis method described in Reference Examples 1 and 2 described later.
In the present invention, the "salt" of the compound (1) represents a salt comprising one acid selected from the group consisting of an inorganic acid, an organic carboxylic acid and an organic sulfonic acid and the compound (1). Here, the "salt" refers to a compound generated by reaction of the compound (1) with a chemically possible number of equivalents of the aforementioned acid and formed of a base positive moiety in the molecule of the compound (1) and an acid negative moiety.

Preferable examples of the inorganic acid salt include hydrofluorides, hydrochlorides, hydrobromides, hydriodides, sulfates, nitrates, perchlorates, phosphates, carbonates, bicarbonates, borates and tetrafluoroborates. More preferable examples of the inorganic acid salt include hydrochlorides, hydrobromides, sulfates, phosphates, perchlorates and tetrafluoroborates.
Preferable examples of the organic carboxylate include acetates, oxalates, maleates, tartrates, fumarates, citrates and malonates. More preferable examples of the organic carboxylate include maleates, tartrates, fumarates and malonates.
Preferable examples of the organic sulfonate include methanesulfonates, trifluoromethanesulfonates, ethanesulfonates, benzenesulfonates, toluenesulfonates and camphorsulfonates. More preferable examples of the organic sulfonate include methanesulfonates and toluenesulfonates.

In the present invention, the solvate of the salt of the compound (1) refers to a solid formed by the salt of the compound (1) together with a solvent molecule. Examples of the solvate include a hydrate formed by the salt of the compound (1) and a water molecule; an alcoholate formed by the salt of the compound (1) and an alcohol molecule such as methanol, ethanol, 1-propanol or isopropanol; a solvate formed by the salt of the compound (1) and a polar solvent such as 1-methyl-2-pyrrolidone, N,N-dimethylformamide or dimethyl sulfoxide; a solvate formed by the salt of the compound (1) and an ester solvent such as ethyl acetate or methyl acetate; and a solvate formed by the salt of the compound (1) and a ketone solvent such as acetone, butanone or cyclohexanone. Among these, a hydrate and an alcoholate are preferable, and a 1-propanolate is particularly preferable.

The salt of the compound (1) or the solvate thereof according to the present invention may be either a crystalline form or an amorphous form.
Preferable specific examples of the salt of the compound (1) or the solvate thereof in the present invention include compound (1) dihydrochloride monohydrate, compound (1) dihydrobromide monohydrate, compound (1) hemisulfate, compound (1) phosphate, compound (1) phosphate 1-propanolate, compound (1) perchlorate, compound (1) tetrafluoroborate, compound (1) monomaleate, compound (1) monotosylate and compound (1) monomesylate.

More specifically, the followings are preferable in the present invention:
crystals of compound (1) dihydrochloride monohydrate having, in powder X-ray diffractometry [diffraction angle (2θ)], a diffraction peak at:
   (1) 10.9°,
   (2) 11.1°,
   (3) 12.7°,
   (4) 13.0°,
   (5) 17.2°,
   (6) 20.7°,
   (7) 24.5° or
   (8) 25.6°, for example;
crystals of compound (1) dihydrobromide monohydrate having, in powder X-ray diffractometry [diffraction angle (2θ)], a diffraction peak at:
   (1) 10.5°,
   (2) 13.2°,
   (3) 17.7°,
   (4) 21.4°,
   (5) 22.3° or
   (6) 39.4°, for example; and
crystals of compound (1) phosphate 1-propanolate having, in powder X-ray diffractometry [diffraction angle (2θ)], a diffraction peak at:
   (1) 8.4°,
   (2) 14.3°,
   (3) 19.3°,
   (4) 21.1° or
   (5) 29.6°, for example,
as representative examples, and various other salt crystals.
Preferable specific examples also include an amorphous form of compound (1) phosphate not having a diffraction peak in powder X-ray diffractometry; an amorphous form of compound (1) monotartrate not having a diffraction peak in powder X-ray diffractometry; an amorphous form of compound (1) hemisulfate not having a diffraction peak in powder X-ray diffractometry; and various other salt amorphous forms.

The aforementioned characteristic peaks in powder X-ray diffractometry are specific to crystals of compound (1) dihydrochloride monohydrate, compound (1) dihydrobromide monohydrate and crystals of compound (1) phosphate 1-propanolate.
Generally, a diffraction angle (2θ) in powder X-ray diffractometry may have an error in the range of ±0.2°. Therefore, the aforementioned diffraction angle values should be understood as including values in the range of about ±0.2°. Accordingly, the present invention includes not only crystals whose peak diffraction angles in powder X-ray diffractometry completely coincide with each other, but also crystals whose peak diffraction angles coincide with each other with an error of about ±0.2°.
Therefore, in the present specification, the phrase "having a diffraction peak at a diffraction angle (2θ ± 0.2°) of 10.9°" means "having a diffraction peak at a diffraction angle (2θ) of 10.7° to 11.1°"; the phrase "having a diffraction peak at a diffraction angle (2θ ± 0.2°) of 11.1°" means "having a diffraction peak at a diffraction angle (2θ) of 10.9° to 11.3°"; the phrase "having a diffraction peak at a diffraction angle (2θ ± 0.2°) of 12.7°" means "having a diffraction peak at a diffraction angle (2θ) of 12.5° to 12.9°"; the phrase "having a diffraction peak at a diffraction angle (2θ ± 0.2°) of 13.0°" means "having a diffraction peak at a diffraction angle (2θ) of 12.8° to 13.2°"; the phrase "having a diffraction peak at a diffraction angle (2θ± 0.2°) of 17.2°" means "having a diffraction peak at a diffraction angle (2θ) of 17.0° to 17.4°"; the phrase "having a diffraction peak at a diffraction angle (2θ ± 0.2°) of 20.7°" means "having a diffraction peak at a diffraction angle (2θ) of 20.5° to 20.9°"; the phrase "having a diffraction peak at a diffraction angle (2θ ± 0.2°) of 24.5°" means "having a diffraction peak at a diffraction angle (2θ) of 24.3° to 24.7°"; the phrase "having a diffraction peak at a diffraction angle (2θ ± 0.2°) of 25.6°" means "having a diffraction peak at a diffraction angle (2θ) of 25.4° to 25.8°"; the phrase "having a diffraction peak at a diffraction angle (2θ ± 0.2°) of 10.5°" means "having a diffraction peak at a diffraction angle (2θ) of 10.3° to 10.7°"; the phrase "having a diffraction peak at a diffraction angle (2θ ± 0.2°) of 13.2°" means "having a diffraction peak at a diffraction angle (2θ) of 13.0° to 13.4°"; the phrase "having a diffraction peak at a diffraction angle (2θ ± 0.2°) of 17.7°" means "having a diffraction peak at a diffraction angle (2θ) of 17.5° to 17.9°"; the phrase "having a diffraction peak at a diffraction angle (2θ ± 0.2°) of 21.4°" means "having a diffraction peak at a diffraction angle (2θ) of 21.2° to 21.6°"; the phrase "having a diffraction peak at a diffraction angle (2θ ± 0.2°) of 22.3°" means "having a diffraction peak at a diffraction angle (2θ) of 22.1° to 22.5°"; the phrase "having a diffraction peak at a diffraction angle (2θ ± 0.2°) of 39.4°" means "having a diffraction peak at a diffraction angle (2θ) of 39.2° to 39.6°"; the phrase "having a diffraction peak at a diffraction angle (2θ ± 0.2°) of 8.4°" means "having a diffraction peak at a diffraction angle (2θ) of 8.2° to 8.6°"; the phrase "having a diffraction peak at a diffraction angle (2θ ± 0.2°) of 14.3°" means "having a diffraction peak at a diffraction angle (2θ) of 14.1° to 14.5°"; the phrase "having a diffraction peak at a diffraction angle (2θ ± 0.2°) of 19.3°" means "having a diffraction peak at a diffraction angle (2θ) of 19.1° to 19.5°"; the phrase "having a diffraction peak at a diffraction angle (2θ ± 0.2°) of 21.1°" means "having a diffraction peak at a diffraction angle (2θ) of 20.9° to 21.3°"; and the phrase "having a diffraction peak at a diffraction angle (2θ ± 0.2°) of 29.6°" means "having a diffraction peak at a diffraction angle (2θ) of 29.4° to 29.8°" .

Next, the method for producing the salt of the compound (1) or the solvate thereof according to the present invention will be described in detail.

### Method for producing salt of compound (1)

The salt of the compound (1) can be obtained by a conventional method for producing a salt. Specifically, for example, the salt can be produced by dissolving the compound (1) in a solvent with heating as necessary; then adding one acid selected from the group consisting of an inorganic acid, an organic carboxylic acid and an organic sulfonic acid to the resulting solution; and stirring the mixture for several minutes to several hours at room temperature or with cooling in an ice bath or leaving the mixture to stand for several minutes to several hours at room temperature or with cooling in an ice bath. The salt of the compound (1) can be obtained as a crystalline form or an amorphous form by this production method. The solvent used herein may be one solvent selected from the group consisting of an alkylketone solvent such as acetone or 2-butanone; ethyl acetate; hexane; acetonitrile; an alcohol solvent such as ethanol, 1-propanol or isopropanol; and water, or a mixed solvent of two or more such solvents, for example. More preferable examples of the solvent include ethyl acetate, acetonitrile, acetone-ethanol, 2-butanone-1-propanol and hexane-acetonitrile.

### Method for producing solvate of salt of compound (1)

The solvate of the salt of the compound (1) can be produced by dissolving the compound (1) in a solvent with heating as necessary; then adding an acid with further addition of a solvent of the solvate to be obtained; and stirring the mixture for several minutes to several hours at room temperature or with cooling in an ice bath or leaving the mixture to stand for several minutes to several hours at room temperature or with cooling in an ice bath in the aforementioned method for producing the salt of the compound (1), for example. In order to obtain a solvate of the solvent first used for dissolving the compound (1), it is not necessary to further add another solvent. The target solvate can be obtained by stirring the mixture or leaving the mixture to stand as is. The solvate of the salt of the compound (1) can be obtained as a crystalline or amorphous form by this production method.

Next, there will be described in detail the methods for producing crystalline forms of compound (1) dihydrochloride monohydrate, compound (1) dihydrobromide monohydrate, compound (1) phosphate 1-propanolate, compound (1) tetrafluoroborate and compound (1) perchlorate as examples and the method for drying the crystalline forms. Crystalline forms other than these can also be produced and dried by the methods described below in detail.

### Method for crystallizing crystals of compound (1) dihydrochloride monohydrate using various recrystallization solvents

The crystals of compound (1) dihydrochloride monohydrate of the present invention can be produced by producing the compound (1) according to the synthesis method described in Reference Example 1 and Reference Example 2; dissolving the compound (1) in a predetermined solvent; adding a hydrochloric acid solution to the solution; leaving the mixture to stand at room temperature; and crystallizing the crystals. Alternatively, the crystals can be produced by similarly producing the compound (1); dissolving the compound (1) in a predetermined solvent; adding a hydrochloric acid solution to the solution; and leaving the mixture to stand at room temperature to once obtain compound (1) dihydrochloride monohydrate; and then dissolving this in a recrystallization solvent to crystallize the crystals.

The method for recrystallizing compound (1) dihydrochloride monohydrate will be described in detail below.
Compound (1) dihydrochloride used for crystallization may be an amorphous form, a crystalline form (including a crystalline form formed of a plurality of crystal polymorphs) or a mixture of these forms.

The solvent used for crystallization may be one solvent selected from the group consisting of an alkylketone solvent such as acetone or 2-butanone; ethyl acetate; hexane; acetonitrile; an alcohol solvent such as ethanol, 1-propanol or isopropanol; and water, or a mixed solvent of two or more such solvents, for example. More preferable examples of the solvent include ethyl acetate, acetonitrile, acetone-ethanol, 2-butanone-1-propanol and hexane-acetonitrile. When compound (1) dihydrochloride used for crystallization is an anhydride, it is necessary to add water to the solvent used for crystallization.
When a mixed solvent such as acetone-ethanol, 2-butanone-1-propanol or hexane-acetonitrile is used, the mixing ratio (by volume) is preferably 20:1 to 1:20, for example, more preferably 10:1 to 1:10, for example, and still more preferably 5:1 to 1:5, for example. The mixed solvent is the most suitably a mixed solvent of acetonitrile-ethanol (5:1), 2-butanone-1-propanol (5:1), acetone-ethanol (10:1) or hexane-acetonitrile (1:1).

The amount of the recrystallization solvent used may be appropriately selected between an amount that allows compound (1) dihydrochloride monohydrate to be dissolved by heating as a lower limit and an amount that does not significantly reduce the yield of the crystals as an upper limit. The amount of the recrystallization solvent used is, based on the volume-to-weight ratio, preferably 5 to 150 times (v/w) the amount of the compound (1), for example, more preferably 5 to 125 times (v/w) the amount of the compound (1), for example, and the most preferably about 100 times the amount of the compound (1) when ethyl acetate is used as the recrystallization solvent, about 10 times the amount of the compound (1) when acetonitrile is used as the solvent, about 10 times the amount of the compound (1) when acetone-ethanol (5:1) is used as the solvent, about 15 times the amount of the compound (1) when 2-butanone-1-propanol (5:1) is used as the solvent, about 10 times the amount of the compound (1) when acetone-ethanol (10:1) is used as the solvent, or about 10 times the amount of the compound (1) when hexane-acetonitrile (1:1) is used as the solvent.

The temperature for dissolving compound (1) dihydrochloride monohydrate by heating may be appropriately selected according to the solvent. The temperature is preferably the reflux temperature of the recrystallization solvent to 15°C, and more preferably 100 to 60°C.
Crystals (polymorphs) having different forms may be provided by changing the cooling rate during crystallization. Therefore, it is preferable to carry out crystallization with the cooling rate appropriately controlled taking an influence on the quality, the grain size and the like of the crystals into consideration. The crystals are preferably cooled at a rate of preferably 40 to 5°C/hour, for example, and more preferably 25 to 15°C/hour, for example.
The final crystallization temperature may be appropriately selected according to the yield, the quality and the like of the crystals and is preferably 30 to -25°C, for example.

Seed crystals (crystals of compound (1) dihydrochloride monohydrate) may or may not be added in crystallization of the crystals. The temperature for addition of the seed crystals is not particularly limited and is preferably 60°C or less, for example, more preferably 55°C to 0°C, for example, still more preferably 55°C to 15°C, for example, and the most preferably about 25°C.

The target crystals can be obtained by separating the crystallized crystals by a conventional filtration operation, washing the separated crystals with a solvent as necessary and further drying the crystals. The solvent used for washing the crystals is the same as the crystallization solvent. Examples of the solvent include ethyl acetate, acetonitrile, 2-butanone-1-propanol (5:1), acetone-ethanol (10:1) and hexane-acetonitrile (1:1).

Similarly, the crystals of compound (1) dihydrochloride monohydrate can be produced by dissolving the compound (1) in a predetermined solvent; adding a hydrochloric acid solution to the solution; and crystallizing the crystals from the mixture.

### Method for crystallizing crystals of compound (1) dihydrobromide monohydrate using various recrystallization solvents

The crystals of compound (1) dihydrobromide monohydrate of the present invention can be produced by producing the compound (1) according to the synthesis method described in Reference Example 1 and Reference Example 2; dissolving the compound (1) in a predetermined solvent; adding a hydrogen bromide solution to the solution; leaving the mixture to stand at room temperature; and crystallizing the crystals. Alternatively, the crystals can be produced by similarly producing the compound (1); dissolving the compound (1) in a predetermined solvent; adding a hydrobromic acid solution to the solution; and leaving the mixture to stand at room temperature to once obtain compound (1) dihydrobromide monohydrate; and then dissolving this in a recrystallization solvent to crystallize the crystals.

The method for recrystallizing compound (1) dihydrobromide monohydrate will be described in detail below.
Compound (1) dihydrobromide used for crystallization may be an amorphous form, a crystalline form (including a crystalline form formed of a plurality of crystal polymorphs) or a mixture of these forms.

The solvent used for crystallization may be preferably one solvent selected from the group consisting of ethyl acetate, hexane, acetonitrile, ethanol, 1-propanol, isopropanol and water, or a mixed solvent of two or more such solvents, for example. More preferable examples of the solvent include ethyl acetate, acetonitrile and ethyl acetate-ethanol.
When a mixed solvent such as ethyl acetate-ethanol is used, the mixing ratio (by volume) is preferably 20:1 to 1:20, for example, and more preferably 5:1 to 1:5, for example. The solvent is the most suitably a mixed solvent of ethyl acetate-ethanol (4.4:1.2).

The amount of the solvent used may be appropriately selected between an amount that allows compound (1) dihydrobromide monohydrate to be dissolved by heating as a lower limit and an amount that does not significantly reduce the yield of the crystals as an upper limit. The amount is, based on the volume-to-weight ratio, preferably 5 to 200 times (v/w) the amount of the compound (1), for example. The amount of the recrystallization solvent is preferably 5 to 150 times (v/w) the amount of the compound (1), for example, and more preferably about 100 times the amount of the compound (1), for example, when ethyl acetate is used as the recrystallization solvent, about 15 times the amount of the compound (1), for example, when acetonitrile is used as the solvent, or about 24.5 times the amount of the compound (1), for example, when ethyl acetate-ethanol (4.4:1.0) is used as the solvent.

The temperature for dissolving compound (1) dihydrobromide monohydrate by heating may be appropriately selected according to the solvent. The temperature is preferably the reflux temperature of the recrystallization solvent to 15°C, and more preferably 100 to 60°C.
Crystals (polymorphs) having different forms may be provided by changing the cooling rate during crystallization. Therefore, it is preferable to carry out crystallization with the cooling rate appropriately controlled taking an influence on the quality, the grain size and the like of the crystals into consideration. The crystals are preferably cooled at a rate of preferably 40 to 5°C/hour, for example, and more preferably 25 to 15°C/hour, for example.
The final crystallization temperature may be appropriately selected according to the yield, the quality and the like of the crystals and is preferably 30 to -25°C, for example.

Seed crystals (crystals of compound (1) dihydrobromide monohydrate) may or may not be added in crystallization of the crystals. The temperature for addition of the seed crystals is not particularly limited and is preferably 60°C or less, for example, more preferably 55°C to 0°C, for example, still more preferably 55°C to 15°C, for example, and the most preferably about 25°C.

The target crystals can be obtained by separating the crystallized crystals by a conventional filtration operation, washing the separated crystals with a solvent as necessary and further drying the crystals. The solvent used for washing the crystals is the same as the crystallization solvent. Preferable examples of the solvent include ethyl acetate, acetonitrile and ethyl acetate-ethanol (4.4:1.0).

### Method for crystallizing crystals of compound (1) phosphate 1-propanolate using various recrystallization solvents

The crystals of compound (1) phosphate 1-propanolate of the present invention can be produced by producing the compound (1) according to the synthesis method described in Reference Example 1 and Reference Example 2; dissolving the compound (1) in propan-1-ol; adding a phosphoric acid solution to the solution; leaving the mixture to stand at room temperature; and crystallizing the crystals. Alternatively, the crystals can be produced by similarly producing the compound (1); dissolving the compound (1) in a predetermined solvent; adding a phosphoric acid solution to the solution; and leaving the mixture to stand at room temperature to once obtain compound (1) phosphate 1-propanolate; and then dissolving this in a recrystallization solvent to crystallize the crystals.

The method for recrystallizing compound (1) phosphate 1-propanolate will be described in detail below.
Compound (1) phosphate 1-propanolate used for crystallization may be an amorphous form, a crystalline form (including a crystalline form formed of a plurality of crystal polymorphs) or a mixture of these forms.

The solvent used for crystallization may be 1-propanol, for example.
The amount of the recrystallization solvent used may be appropriately selected between an amount that allows compound (1) phosphate 1-propanolate to be dissolved by heating as a lower limit and an amount that does not significantly reduce the yield of the crystals as an upper limit. The amount of the recrystallization solvent used is, based on the volume-to-weight ratio, preferably 5 to 150 times (v/w) the amount of the compound (1), for example, more preferably 5 to 125 times (v/w) the amount of the compound (1), for example, and the most preferably about 20 times the amount of the compound (1), for example.

The temperature for dissolving compound (1) phosphate 1-propanolate by heating may be appropriately selected according to the solvent. The temperature is preferably the reflux temperature of the recrystallization solvent to 15°C, and more preferably 100 to 20°C.
Crystals (polymorphs) having different forms may be provided by changing the cooling rate during crystallization. Therefore, it is preferable to carry out crystallization with the cooling rate appropriately controlled taking an influence on the quality, the grain size and the like of the crystals into consideration. The crystals are preferably cooled at a rate of preferably 40 to 5°C/hour, for example, and more preferably 25 to 15°C/hour, for example.
The final crystallization temperature may be appropriately selected according to the yield, the quality and the like of the crystals and is preferably 30 to -25°C, for example.

Seed crystals (crystals of compound (1) phosphate 1-propanolate) may or may not be added in crystallization of the crystals. The temperature for addition of the seed crystals is not particularly limited and is preferably 60°C or less, for example, more preferably 55°C to 0°C, for example, still more preferably 55°C to 15°C, for example, and the most preferably about 25°C.

The target crystals can be obtained by separating the crystallized crystals by a conventional filtration operation, washing the separated crystals with a solvent as necessary and further drying the crystals. The solvent used for washing the crystals is the same as the crystallization solvent and is preferably 1-propanol, for example.
Similarly, the crystals of compound (1) phosphate 1-propanolate can be produced by dissolving the compound (1) in a predetermined solvent; adding a phosphoric acid solution to the solution; and crystallizing the crystals from the mixture.

### Method for crystallizing crystals of compound (1) tetrafluoroborate using various recrystallization solvents

The crystals of compound (1) tetrafluoroborate of the present invention can be produced by producing the compound (1) according to the synthesis method described in Reference Example 1 and Reference Example 2; dissolving the compound (1) in a predetermined solvent; adding tetrafluoroboric acid to the solution; cooling the mixture in an ice bath; and crystallizing the crystals. Alternatively, the crystals can be produced by similarly producing the compound (1); dissolving the compound (1) in a predetermined solvent; adding tetrafluoroboric acid to the solution; and cooling the mixture in an ice bath to once obtain compound (1) tetrafluoroborate; and then dissolving this in a recrystallization solvent to crystallize the crystals.

The method for recrystallizing compound (1) tetrafluoroborate will be described in detail below.
Compound (1) tetrafluoroborate used for crystallization may be an amorphous form, a crystalline form (including a crystalline form formed of a plurality of crystal polymorphs) or a mixture of these forms.

The solvent used for crystallization may be one solvent selected from the group consisting of an alkylketone solvent such as acetone or 2-butanone; ethyl acetate; hexane; acetonitrile; an alcohol solvent such as ethanol, 1-propanol or isopropanol; and water, or a mixed solvent of two or more such solvents, for example. The solvent is more preferably ethyl acetate, for example.
Solvent evaporation using a mixed solvent may be used.

The amount of the recrystallization solvent used may be appropriately selected between an amount that allows compound (1) tetrafluoroborate to be dissolved by heating as a lower limit and an amount that does not significantly reduce the yield of the crystals as an upper limit. The amount of the recrystallization solvent used is, based on the volume-to-weight ratio, preferably 5 to 150 times (v/w) the amount of the compound (1), for example, more preferably 5 to 125 times (v/w) the amount of the compound (1), for example, and the most preferably about 25 times the amount of the compound (1), for example, when ethyl acetate is used as the recrystallization solvent.

The temperature for dissolving compound (1) tetrafluoroborate by heating may be appropriately selected according to the solvent. The temperature is preferably the reflux temperature of the recrystallization solvent to 15°C, and more preferably 100 to 20°C.
Crystals (polymorphs) having different forms may be provided by changing the cooling rate during crystallization. Therefore, it is preferable to carry out crystallization with the cooling rate appropriately controlled taking an influence on the quality, the grain size and the like of the crystals into consideration. The crystals are preferably cooled at a rate of preferably 40 to 5°C/hour, for example, and more preferably 25 to 15°C/hour, for example.
The final crystallization temperature may be appropriately selected according to the yield, the quality and the like of the crystals and is preferably 30 to -25°C, for example.

Seed crystals (crystals of compound (1) tetrafluoroborate) may or may not be added in crystallization of the crystals. The temperature for addition of the seed crystals is not particularly limited. The temperature is preferably 60°C or less, for example, more preferably 55°C to 0°C, for example, still more preferably 55°C to 15°C, for example, and the most preferably about 25°C.

The target crystals can be obtained by separating the crystallized crystals by a conventional filtration operation, washing the separated crystals with a solvent as necessary and further drying the crystals. The solvent used for washing the crystals is the same as the crystallization solvent and is preferably ethyl acetate, for example.

Similarly, the crystals of compound (1) tetrafluoroborate can be produced by dissolving the compound (1) in a predetermined solvent; adding tetrafluoroboric acid to the solution; and crystallizing the crystals from the mixture.

### Method for crystallizing crystals of compound (1) perchlorate using various recrystallization solvents

The crystals of compound (1) perchlorate of the present invention can be produced by producing the compound (1) according to the synthesis method described in Reference Example 1 and Reference Example 2; dissolving the compound (1) in a predetermined solvent; adding perchloric acid to the solution; cooling the mixture in an ice bath; and crystallizing the crystals. Alternatively, the crystals can be produced by similarly producing the compound (1); dissolving the compound (1) in a predetermined solvent; adding perchloric acid to the solution; and cooling the mixture in an ice bath to once obtain compound (1) perchlorate; and then dissolving this in a recrystallization solvent to crystallize the crystals.

The method for recrystallizing compound (1) perchlorate will be described in detail below.
Compound (1) perchlorate used for crystallization may be an amorphous form, a crystalline form (including a crystalline form formed of a plurality of crystal polymorphs) or a mixture of these forms.

The solvent used for crystallization may be one solvent selected from the group consisting of an alkylketone solvent such as acetone or 2-butanone; ethyl acetate; hexane; acetonitrile; an alcohol solvent such as ethanol, 1-propanol or isopropanol; and water, or a mixed solvent of two or more such solvents, for example. The solvent is more preferably ethyl acetate, for example.
Solvent evaporation using a mixed solvent may be used.

The amount of the recrystallization solvent used may be appropriately selected between an amount that allows compound (1) perchlorate to be dissolved by heating as a lower limit and an amount that does not significantly reduce the yield of the crystals as an upper limit. The amount of the recrystallization solvent used is, based on the volume-to-weight ratio, preferably 5 to 150 times (v/w) the amount of the compound (1), for example, more preferably 5 to 125 times (v/w) the amount of the compound (1), for example, and the most preferably about 20 times the amount of the compound (1), for example, when ethyl acetate is used as the recrystallization solvent.

The temperature for dissolving compound (1) perchlorate by heating may be appropriately selected according to the solvent. The temperature is preferably the reflux temperature of the recrystallization solvent to 15°C, and more preferably 100 to 20°C.
Crystals (polymorphs) having different forms may be provided by changing the cooling rate during crystallization. Therefore, it is preferable to carry out crystallization with the cooling rate appropriately controlled taking an influence on the quality, the grain size and the like of the crystals into consideration. The crystals are preferably cooled at a rate of preferably 40 to 5°C/hour, for example, and more preferably 25 to 15°C/hour, for example.
The final crystallization temperature may be appropriately selected according to the yield, the quality and the like of the crystals and is preferably 30 to -25°C, for example.

Seed crystals (crystals of compound (1) perchlorate) may or may not be added in crystallization of the crystals. The temperature for addition of the seed crystals is not particularly limited and is preferably 60°C or less, for example, more preferably 55°C to 0°C, for example, still more preferably 55°C to 15°C, for example, and the most preferably about 25°C.

The target crystals can be obtained by separating the crystallized crystals by a conventional filtration operation, washing the separated crystals with a solvent as necessary and further drying the crystals. The solvent used for washing the crystals is the same as the crystallization solvent and is preferably ethyl acetate, for example.

Similarly, the crystals of compound (1) perchlorate can be produced by dissolving the compound (1) in a predetermined solvent; adding perchloric acid to the solution; and crystallizing the crystals from the mixture.

### Method for drying crystals

The crystals separated by a filtration operation as described above can be appropriately dried by leaving the crystals to stand in the air or heating the crystals.
The drying time may be appropriately selected as a time until the amount of the residual solvent is below a predetermined amount according to the production amount, the drying device, the drying temperature and the like. The drying can be carried out under ventilation or under reduced pressure.
The degree of pressure reduction may be appropriately selected according to the production amount, the drying device, the drying temperature and the like. The resulting crystals may be left to stand in the air as necessary after drying.

The crystals of compound (1) dihydrochloride monohydrate, compound (1) dihydrobromide monohydrate, compound (1) phosphate 1-propanolate, compound (1) tetrafluoroborate or compound (1) perchlorate obtained by the aforementioned method are formed of a single crystal form. The crystal form is stable, is not easily transformed into another crystal form or amorphous form, has excellent properties and is also suitable for formulation.

Next, the production method and the drying method for the amorphous form of the salt of the compound (1) or the solvate thereof according to the present invention will be described in detail.

### Method for producing amorphous form

The amorphous form of the salt of the compound (1) with one acid selected from the group consisting of an inorganic acid, an organic carboxylic acid and an organic sulfonic acid according to the present invention is produced using a general method for producing an amorphous form. Specifically, the amorphous form can be produced by dissolving the compound (1) produced according to the synthesis method described in Reference Example 1 and Reference Example 2 in a solvent with heating as necessary; then adding the aforementioned acid to the solution; stirring the mixture for several minutes to several hours at room temperature or with cooling in an ice bath or leaving the mixture to stand for several minutes to several hours at room temperature or with cooling in an ice bath; and thereafter evaporating the solvent under reduced pressure.

The solvent used may be one solvent selected from the group consisting of an alkylketone solvent such as acetone or 2-butanone; ethyl acetate; hexane; acetonitrile; an alcohol solvent such as ethanol, 1-propanol or isopropanol; N,N-dimethylformamide; and water, or a mixed solvent of two or more such solvents, for example. More preferable examples of the solvent include ethyl acetate, acetonitrile, methanol and ethanol.

The amount of the solvent used may be appropriately selected with an amount that allows the compound (1) to be dissolved by heating as a lower limit and is preferably, based on the volume-to-weight ratio, 5 to 100 times (v/w) the amount of the compound (1), for example. The amount of the solvent used is preferably 5 to 60 times (v/w) the amount of the compound (1), for example, and more preferably about 50 times the amount of the compound (1), for example, when ethyl acetate is used as the solvent.

The temperature for dissolving the compound (1) by heating may be appropriately selected according to the solvent and is preferably 15°C to the reflux temperature of the solvent, for example, and more preferably room temperature to 60°C, for example.

Similarly, the amorphous form of the salt of the compound (1) which has excellent solubility, is not easily transformed into a crystal form and has excellent properties can be obtained by lyophilization using one solvent selected from the group consisting of dimethylformamide, dimethyl sulfoxide and water or a mixed solvent of two or more such solvents, for example.

### Method for drying amorphous form

The amorphous form obtained as described above can be appropriately dried by leaving the amorphous form to stand in the air or heating the amorphous form.
The drying time may be appropriately selected as a time until the amount of the residual solvent is below a predetermined amount according to the production amount, the drying device, the drying temperature and the like. The drying can be carried out under ventilation or under reduced pressure.
The degree of pressure reduction may be appropriately selected according to the production amount, the drying device, the drying temperature and the like. The resulting amorphous form may be left to stand in the air as necessary after drying.

The amorphous form of the salt of the compound (1) obtained by the aforementioned method is stable, is not easily transformed into a crystal form, has excellent properties and is also suitable for formulation.

The compound (1) has an effect of reducing Aβ production and can be used as an active ingredient in a therapeutic agent for a neurodegenerative disease caused by Aβ such as Alzheimer's disease or Down's syndrome.

The salt of the compound (1) or the solvate thereof according to the present invention used as a drug is orally or parenterally administered as a therapeutic agent for a neurodegenerative disease caused by Aβ such as Alzheimer's disease or Down's syndrome, for example. The dose varies according to the degree of symptom, the age, sex and weight of the patient, the difference in sensitivity among the patients, the administration method, the administration period, the administration interval, the character of the pharmaceutical preparation, the formulation, the type, and the type of the active ingredient, for example, and is not particularly limited. The dose is usually 100 to 6000 mg per adult per day, preferably about 50 to 4000 mg per adult per day, and still more preferably about 100 to 3000 mg per adult per day, for example, and is usually administered in one to three divided doses per day.

An oral solid preparation is prepared by adding an excipient and, as necessary, a binder, a disintegrant, a lubricant, a colorant, a corrective and the like to the principal agent, and then formulating tablets, coated tablets, granules, fine granules, powder or capsules, for example, by a conventional method. Examples of the expicient used include lactose, corn starch, sucrose, glucose, sorbitol, crystalline cellulose and silicon dioxide. Examples of the binder used include polyvinyl alcohol, ethylcellulose, methylcellulose, gum arabic, hydroxypropylcellulose and hydroxypropylmethylcellulose. Examples of the lubricant used include magnesium stearate, talc and silica. Examples of the colorant used include those permitted to be added to pharmaceuticals. Examples of the corrective used include cacao powder, menthol, aromatic acid, peppermint oil, borneol and cinnamon powder. These tablets or granules may be appropriately coated with sugar, gelatin or other coatings as necessary, obviously. An injection is prepared by adding a pH adjuster, a buffer, a suspending agent, a solubilizer, a stabilizer, an isotonizing agent and a preservative, for example, to the principal agent as necessary and formulating an intravenous, subcutaneous or intramuscular injection, for example, by a conventional method. In this case, the injection may be formulated as a lyophilized product by a conventional method. Examples of the suspending agent include methylcellulose, polysolvate 80, hydroxyethylcellulose, gum arabic, tragacanth powder, sodium carboxymethylcellulose and polyoxyethylene sorbitan monolaurate.

According to the present invention, crystals of compound (1) dihydrochloride monohydrate can be obtained as a single crystal form using acetonitrile, acetone-ethanol (5:1), 2-butanone-1-propanol (5:1), acetone-ethanol (10:1) or hexane-acetonitrile (1:1) as a recrystallization solvent. Further, crystals of compound (1) dihydrobromide monohydrate can be obtained as a single crystal form using ethyl acetate, acetonitrile or ethyl acetate-ethanol (4.4:1.0) as a recrystallization solvent. Similarly, crystals of compound (1) phosphate 1-propanolate, crystals of compound (1) tetrafluoroborate and crystals of compound (1) perchlorate can be obtained as single crystal forms.
Further, amorphous forms of compound (1) monofumarate, compound (1) monotartrate, compound (1) monomaleate, compound (1) hemisulfate, compound (1) monotosylate and compound (1) monomesylate can be obtained by mixing the compound (1) with an acid in an appropriate solvent and then evaporating the solvent, respectively.
These are salts of the compound (1) suitable for pharmaceutical preparations, solvates thereof, crystal polymorphs thereof and amorphous forms thereof.

### BEST MODE FOR CARRYING OUT THE INVENTION

The present invention will be described in detail below with reference to reference examples and examples; however, the present invention is not limited to these examples.

The following abbreviations are used in the following examples.
DMF: N,N-Dimethylformamide
THF: Tetrahydrofuran
EDC: 1-Ethyl-3-(3-dimethylaminopropyl)carbodiimide hydrochloride
HOBT: 1-Hydroxybenzotriazole
IPEA: Diisopropylethylamine

### Reference Example 1

### Synthesis of (3E)-1-[(1S)-1-(4-fluorophenyl)ethyl]-3-[3-methoxy-4-(4-methyl-1H-imidazol-1-yl)benzylidene]piperidin-2-one

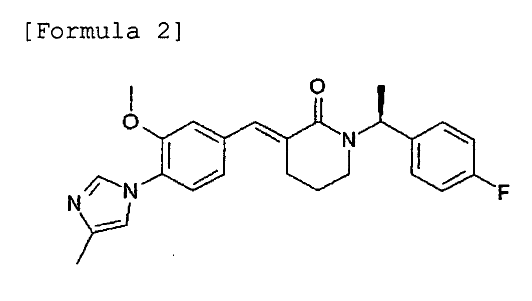

### Synthesis of 5-chloro-2-(diethoxyphosphoryl)valeric acid tert-butyl ester

Sodium hydride (containing 40% mineral oil, 17.4 g) was washed with hexane (100 mL) three times to remove the oil. A solution of diethylphosphonoacetic acid tert-butyl ester (100 g) in THF (100 mL) was added dropwise to a suspension of the sodium hydride in THF (500 mL) at 0°C over 30 minutes. Then, the reaction solution was heated to room temperature and further stirred for one hour. A solution of 1-bromo-3-chloropropane (125 g) in THF (100 mL) was added dropwise to the reaction solution over 30 minutes. After completion of the dropwise addition, the reaction solution was heated under reflux for 15 hours. The reaction solution was allowed to cool to room temperature. Ethyl acetate (1 L) and saturated aqueous ammonium chloride (1 L) were added and the organic layer was separated. The resulting organic layer was dried over anhydrous magnesium sulfate and concentrated under reduced pressure to provide 113.4 g of the title compound. The property values of the compound are as follows.
¹H-NMR (CDCl₃) δ(ppm): 1.31-1.48 (m, 6H), 1.48 (s, 9H), 1.79-2.14 (m, 4H), 2.73-2.91 (m, 1H), 3.55 (t, J = 6.4 Hz, 2H), 4.10-4.19 (m, 4H).

### Synthesis of (3E)-5-chloro-2-[3-methoxy-4-(4-methyl-1H-imidazol-1-yl)benzylidene]valeric acid tert-butyl ester

5-Chloro-2-(diethoxyphosphoryl)valeric acid tert-butyl ester (83.5 g) and lithium hydroxide monohydrate (29.1 g) were sequentially added to a solution of 3-methoxy-4-(4-methyl-1H-imidazol-1-yl)benzaldehyde obtained in Reference Example 2 (50 g) in THF (600 mL) and ethanol (200 mL). The reaction solution was stirred at room temperature overnight. After confirming disappearance of the raw materials, water and ethyl acetate were added to the reaction solution and the organic layer was separated. The resulting organic layer was washed with brine and then dried over anhydrous magnesium sulfate and concentrated under reduced pressure. The residue was purified by silica gel chromatography (elution solvent: heptane:ethyl acetate = 1:1) and the resulting solid was recrystallized from a mixed solution of ethyl acetate and hexane to provide 54.9 g of the title compound. The property values of the compound are as follows.
¹H-NMR (CDCl₃) δ(ppm): 1.55 (s, 9H), 1.99-2.08 (m, 2H), 2.30 (s, 3H), 2.63-2.71 (m, 2H), 3.59 (t, J = 6.4 Hz, 2H), 3.87 (s, 3H), 6.93 (m, 1H), 7.00 (d, J = 1.2 Hz, 1H), 7.09 (dd, J = 8.4, 1.2 Hz, 1H), 7.27 (d, J = 8.4 Hz, 1H), 7.58 (s, 1H), 7.72 (m, 1H).

### Synthesis of (3E)-5-chloro-2-[3-methoxy-4-(4-methyl-1H-imidazol-1-yl)benzylidenelvaleric acid trifluoroacetate

Trifluoroacetic acid (10 mL) was added to a solution of (3E)-5-chloro-2-[3-methoxy-4-(4-methyl-1H-imidazol-1-yl)benzylidene]valeric acid tert-butyl ester (5 g) in methylene chloride (20 mL). The reaction solution was stirred at room temperature for two hours. After confirming disappearance of the raw materials, the reaction solution was concentrated under reduced pressure. The resulting solid was collected by filtration and washed with ethyl acetate to provide 5.7 g of the title compound. The property values of the compound are as follows.
¹H-NMR (DMSO-d₆) δ(ppm): 1.93-2.03 (m, 2H), 2.35 (s, 3H), 2.58-2.66 (m, 2H), 3.70 (t, J = 6.4 Hz, 2H), 3.91 (s, 3H), 7.24 (dd, J = 8.4, 1.2 Hz, 1H), 7.37 (d, J = 1.2 Hz, 1H), 7.64 (d, J = 8.4, 1H), 7.66 (m, 1H), 7.76 (s, 1H), 9.36 (m, 1H).

### Synthesis of (3E)-5-chloro-2-[3-methoxy-4-(4-methyl-1H-imidazol-1-yl)benzylidene]valeric acid [(S)-1-(4-fluorophenyl)ethyl]amide

IPEA (12.4 mL), EDC (6.82 g) and HOBT (4.81 g) were sequentially added to a solution of the resulting (3E)-5-chloro-2-[3-methoxy-4-(4-methyl-1H-imidazol-1-yl)benzylidene]valeric acid trifluoroacetate (8.00 g) and (S)-1-(4-fluorophenyl)ethylamine (2.60 g) in DMF (50 mL). The reaction solution was stirred at room temperature overnight. After confirming disappearance of the raw materials, the solvent was concentrated under reduced pressure. Water and ethyl acetate were added to the residue and the organic layer was separated. The organic layer was washed with brine and then dried over anhydrous magnesium sulfate and concentrated under reduced pressure. The resulting residue was purified by silica gel chromatography (eluent solvent: heptane:ethyl acetate = 2:3 -> 1:1 -> ethyl acetate) to provide 3.90 g of the title compound. The property values of the compound are as follows.
¹H-NMR (CDCl₃) δ(ppm): 1.56 (d, J = 6.8 Hz, 3H), 1.95-2.02 (m, 2H), 2.30 (s, 3H), 2.70-2.74 (m, 2H), 3.58 (t, J = 6.0 Hz, 2H), 3.85 (s, 3H), 5.17-5.24 (m, 1H), 6.15 (d, J = 6.8 Hz, 1H), 6.92-6.96 (m, 3H), 7.02-7.07 (m, 2H), 7.17 (s, 1H), 7.23-7.25 (m, 1H), 7.32-7.36 (m, 2H), 7.70-7.71 (s, 1H).

### Synthesis of (3E)-1-[(S)-1-(4-fluorophenyl)ethyl]-3-[3-methoxy-4-(4-methyl-1H-imidazol-1-yl)benzylidene]piperidin-2-one

Sodium hydride (containing 40% mineral oil, 410 mg) was added to a solution of (3E)-5-chloro-2-[3-methoxy-4-(4-methyl-1H-imidazol-1-yl)benzylidene]valeric acid [(S)-1-(4-fluorophenyl)ethyl]amide (3.90 g) in DMF (30 mL) at 0°C. Then, the reaction solution was heated to room temperature and stirred overnight. After confirming disappearance of the raw materials, the reaction solution was cooled to 0°C. Water and ethyl acetate were added to the reaction solution and the organic layer was separated. The resulting organic layer was washed with brine and then dried over anhydrous magnesium sulfate and concentrated under reduced pressure. The residue was purified by silica gel chromatography (elution solvent: ethyl acetate -> ethyl acetate:ethanol 10:1). The resulting solid was washed with diethyl ether and further recrystallized from ethyl acetate to provide 2.60 g of the title compound. The property values of the compound are as follows. ¹H-NMR (CDCl₃) δ(ppm): 1.50 (d, J = 7.2 Hz, 3H), 1.65-1.74 (m, 1H), 1.78-1.87 (m, 1H), 2.30 (s, 3H), 2.71-2.85 (m, 2H), 2.91-2.97 (m, 1H), 3.24 (ddd, J = 3.6, 8.8, 12.0 Hz, 1H), 3.86 (s, 3H), 6.23 (q, J = 7.2 Hz, 1H), 6.93 (t, J = 1.2 Hz, 1H), 7.00-7.06 (m, 4H), 7.24-7.26 (m, 1H), 7.31-7.34 (m, 2H), 7.72 (d, J = 1.2 Hz, 1H), 7.89 (s, 1H).

### Reference Example 2

### Synthesis of 3-methoxy-4-(4-methyl-1H-imidazol-1-yl)benzaldehyde

### Synthesis of 3-methoxy-4-(4-methyl-1H-imidazol-1-yl)benzaldehyde and 3-methoxy-4-(5-methyl-1H-imidazol-1-yl)benzaldehyde

Potassium carbonate (4.05 g) was added to a solution of 4-fluoro-3-methoxybenzaldehyde (3.00 g) and 4-methylimidazole (3.307 g) in DMF (50 mL) and the reaction solution was stirred at 100°C overnight. The resulting reaction mixture was concentrated under reduced pressure. Water and ethyl acetate were added to the residue and the organic layer was separated. The organic layer was washed with brine and then dried over anhydrous magnesium sulfate and concentrated under reduced pressure. The residue was purified by silica gel column chromatography (elution solvent: hexane-ethyl acetate system) to provide 3-methoxy-4-(4-methyl-1H-imidazol-1-yl)benzaldehyde (856 mg) and 3-methoxy-4-(5-methyl-1H-imidazol-1-yl)benzaldehyde (44 mg).
The property values of 3-methoxy-4-(4-methyl-1H-imidazol-1-yl)benzaldehyde are as follows.
¹H-NMR (CDCl₃) δ(ppm): 2.31 (s, 3H), 3.97 (s, 3H), 7.02 (brs, 1H), 7.44 (d, J = 8.0 Hz, 1H), 7.55 (dd, J = 1.6 Hz, 8.0 Hz, 1H), 7.58 (d, J = 1.6 Hz, 1H), 7.84 (brs, 1H), 10.00 (s, 1H),
The property values of 3-methoxy-4-(5-methyl-1H-imidazol-1-yl)benzaldehyde are as follows.
¹H-NMR (CDCl₃ ) δ(ppm) : 2.10 (s, 3H), 3.90 (s, 3H), 6.91 (brs, 1H), 7.40 (d, J = 8.0 Hz, 1H), 7.50 (d, J = 1.2 Hz, 1H), 7.57-7.59 (m, 1H), 7.84 (s, 1H), 10.05 (s, 1H),
3-Methoxy-4-(4-methyl-1H-imidazol-1-yl)benzaldehyde can also be separately synthesized by the following method.

### Synthesis of 3-methoxy-4-nitrobenzoic acid methyl ester

Methyl iodide (463 g) was added dropwise to a mixture of 3-hydroxy-4-nitrobenzoic acid (199 g) and potassium carbonate (450 g) in DMF (1 L) at room temperature. The reaction solution was stirred at room temperature overnight and then methyl iodide (230 g) was added to the reaction solution. The reaction solution was further stirred at room temperature for six hours. The reaction solution was added to ice water and the precipitated solid was collected by filtration. The resulting solid was dried at 50°C overnight to provide 178 g of the title compound. The property values corresponded to the reported values (CAS #5081-37-8).

### Synthesis of 4-amino-3-methoxybenzoic acid methyl ester

10% palladium-carbon (containing 50% water, 15 g) was added to a solution of 3-methoxy-4-nitrobenzoic acid methyl ester (150 g) in methanol (600 mL) and THF (300 mL) and the reaction solution was stirred at a hydrogen pressure of 0.9 MPa at 50°C to 64°C for 6.5 hours. The reaction solution was allowed to cool to room temperature and then filtered through celite. The resulting filtrate was concentrated under reduced pressure to provide 134 g of the title compound. The property values corresponded to the reported values (CAS #41608-64-4).

### Synthesis of 4-formylamino-3-methoxybenzoic acid methyl ester

Acetic anhydride (268 mL) was added dropwise to formic acid (401 mL) at room temperature and the reaction solution was stirred at room temperature for 40 minutes. A solution of 4-amino-3-methoxybenzoic acid methyl ester (134 g) in THF (600 mL) was added dropwise to the reaction solution at room temperature and the reaction solution was stirred for one hour. To the reaction solution was added 3.8 L of ice water, and the precipitated solid was filtered and further washed with water (2 L). The resulting solid was dried at 50°C overnight to provide 111 g of the title compound. The property values corresponded to the reported values (CAS #700834-18-0).

### Synthesis of 4-[formyl-(2-oxopropyl)amino]-3-methoxybenzoic acid methyl ester

Chloroacetone (84.5 mL) was added dropwise to a mixture of 4-formylamino-3-methoxybenzic acid methyl ester (111 g), cesium carbonate (346 g) and potassium iodide (8.78 g) in DMF (497 mL) at room temperature and the reaction solution was stirred for three hours. Cesium carbonate (173 g) and chloroacetone (42.0 mL) were added to the reaction solution, which was then stirred at room temperature for two hours. Ice water and ethyl acetate were added to the reaction solution and the organic layer was separated.
Ethyl acetate was added to the aqueous layer and the organic layer was separated. The organic layers were combined and washed with water and brine in this order. The resulting organic layers were dried over anhydrous magnesium sulfate and then concentrated under reduced pressure. The residue was diluted with toluene and the solution was concentrated under reduced pressure. tert-Butyl methyl ether and heptane were added to the resulting residue. The precipitated solid was collected by filtration and washed with a solution of 50% tert-butyl methyl ether in heptane. The resulting solid was air-dried overnight to provide 118 g of the title compound.
¹H-NMR (CDCl₃) δ(ppm): 2.19 (s, 3H), 3.91 (s, 3H), 3.94 (s, 3H), 4.49 (s, 2H), 7.31 (d, J = 8.0 Hz, 1H), 7.63 (d, J = 2.0 Hz, 1H), 7.69 (dd, J = 8.0, 2.0 Hz, 1H), 8.33 (s, 1H).

### Synthesis of 3-methoxy-4-(4-methyl-1H-imidazol-1-yl)benzoic acid methyl ester

A solution of 4-[formyl-(2-oxopropyl)amino]-3-methoxybenzoic acid methyl ester (118 g) and ammonium acetate (172 g) in acetic acid (255 mL) was heated and stirred at 140°C for one hour. After completion of the reaction, the reaction solution was neutralized with aqueous ammonia under ice-cooling. Ethyl acetate was added to the reaction solution and the organic layer was separated. The resulting organic layer was dried over anhydrous magnesium sulfate and then filtered on a silica gel pad. The filtrate was concentrated under reduced pressure. tert-Butyl methyl ether and heptane were added to the residue. The precipitated solid was collected by filtration and washed with a solution of 50% tert-butyl methyl ether in heptane. The resulting solid was air-dried overnight to provide 68.4 g of the title compound. Further, the crystallization mother liquor was concentrated under reduced pressure. The residue was purified by silica gel column chromatography (elution solvent: heptane-ethyl acetate system) to provide 22.3 g of the title compound.
¹H-NMR (CDCl₃ ) δ (ppm): 2.30 (s, 3H), 3.94 (s, 3H), 3.96 (s, 3H), 6.98 (brs, 1H), 7.32 (d, J = 8.4 Hz, 1H), 7.71-7.73 (m, 2H), 7.79 (brs, 1H).

### Synthesis of 3-methoxy-4-(4-methyl-1H-imidazol-1-yl)benzaldehyde

A solution of pyrrolidine (18 mL) in THF (45 mL) was added dropwise to a solution of sodium bis(2-methoxyethoxy)aluminum hydride (65% solution in toluene, 56 mL) in THF (60 mL) at -5°C or less over 15 minutes. The reaction solution was stirred at room temperature for one hour. Then, a suspension of tert-butoxide (2.10 g) in THF (15 mL) was added dropwise to the reaction solution at room temperature and the reaction solution was stirred for 15 minutes. The above reaction solution was added dropwise to a solution of 3-methoxy-4-(4-methyl-1H-imidazol-1-yl)benzoic acid methyl ester (20 g) in THF (50 mL) under ice-cooling over 30 minutes. The reaction solution was stirred at room temperature for two hours and then a 5 N sodium hydroxide solution (150 mL) was added dropwise to the reaction solution. Ethyl acetate was added to the reaction solution and the organic layer was separated. The organic layer was washed with a saturated ammonium chloride solution and brine in this order.
The organic layer was dried over anhydrous magnesium sulfate and filtered on a silica gel pad. Then, the filtrate was concentrated under reduced pressure. The residue was diluted with ethyl acetate and the precipitated solid was collected by filtration. The resulting solid was air-dried overnight to provide 7.10 g of the title compound. Further, the crystallization mother liquor was concentrated under reduced pressure. The residue was purified by silica gel column chromatography (elution solvent: heptane-ethyl acetate-2-propanol system) to provide 2.65 g of the title compound.

### Example 1

### Synthesis of (3E)-1-[(1S)-1-(4-fluorophenyl)ethyl]-3-[3-methoxy-4-(4-methyl-1H-imidazol-1-yl)benzylidene]piperidin-2-one dihydrochloride monohydrate

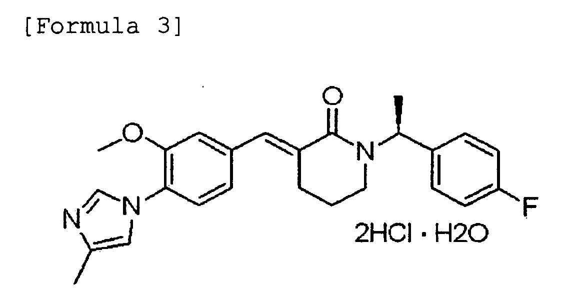

Hydrochloric acid (37%, 11.8 µL) dissolved in ethyl acetate (1 mL) was added to a solution of (3E)-1-[(1S)-1-(4-fluorophenyl)ethyl]-3-[3-methoxy-4-(4-methyl-1H-imidazol-1-yl)benzylidene]piperidin-2-one (20 mg) in ethyl acetate (1 mL). The reaction mixture was stirred at room temperature. The precipitated solid was separated by filtration and washed with ethyl acetate to provide 20 mg of the title compound. The property values of the compound are as follows.

¹H-NMR (CD₃OD δ(ppm) : 1.58 (d, J = 7.2 Hz, 3H), 1.65-1.74 (m, 1H), 1.80-1.89 (m, 1H), 2.43 (d, J = 0.8 Hz, 3H), 2.80-2.84 (m, 2H), 2.99 (ddd, J = 4.4, 6.4, 12.4 Hz, 1H), 3.37 (ddd, J = 12.4, 8.4, 3.6 Hz, 1H), 3.95 (s, 3H), 6.09 (q, J = 7.2 Hz, 1H), 7.07-7.12 (m, 2H), 7.21 (dd, J = 8.0, 1.2 Hz, 1H), 7.31 (d, J = 1.2 Hz, 1H), 7.36-7.40 (m, 2H), 7.57 (d, J = 8.0 Hz, 1H), 7.60 (t, J = 1.2 Hz, 1H), 7.79-7.81 (m, 1H) , 9. 17 (d, J = 1.2 Hz, 1H) .

### Example 2

### Crystallization of (3E)-1-[(1S)-1-(4-fluorophenyl)ethyl]-3-[3-methoxy-4-(4-methyl-1H-imidazol-1-yl)benzylidene]piperidin-2-one dihydrochloride monohydrate

A suspension of (3E)-1-[(1S)-1-(4-fluorophenyl)ethyl]-3-[3-methoxy-4-(4-methyl-1H-imidazol-1-yl)benzylidene]piperidin-2-one dihydrochloride monohydrate (100 mg) in acetonitrile (1 mL) was heated to 80°C and the compound was completely dissolved. Then, the solution was gradually cooled overnight. The precipitated solid was separated by filtration and washed with acetonitrile to provide 47 mg of a crystalline form.

### Example 3

### Crystallization of (3E)-1-[(1S)-1-(4-fluorophenyl)ethyl]-3-[3-methoxy-4-(4-methyl-1H-imidazol-1-yl)benzylidene]piperidin-2-one dihydrochloride monohydrate

### (1) Crystallization method

A suspension of (3E)-1-[(1S)-1-(4-fluorophenyl)ethyl]-3-[3-methoxy-4-(4-methyl-1H-imidazol-1-yl)benzylidene]piperidin-2-one dihydrochloride monohydrate (534 mg) in acetone-ethanol (5:1, 5 mL) was refluxed and the compound was completely dissolved. Then, the solution was gradually cooled overnight. The precipitated solid was separated by filtration and washed with acetone-ethanol (5:1) to provide 280 mg of a crystalline form.

### (2) Powder X-ray diffractometry

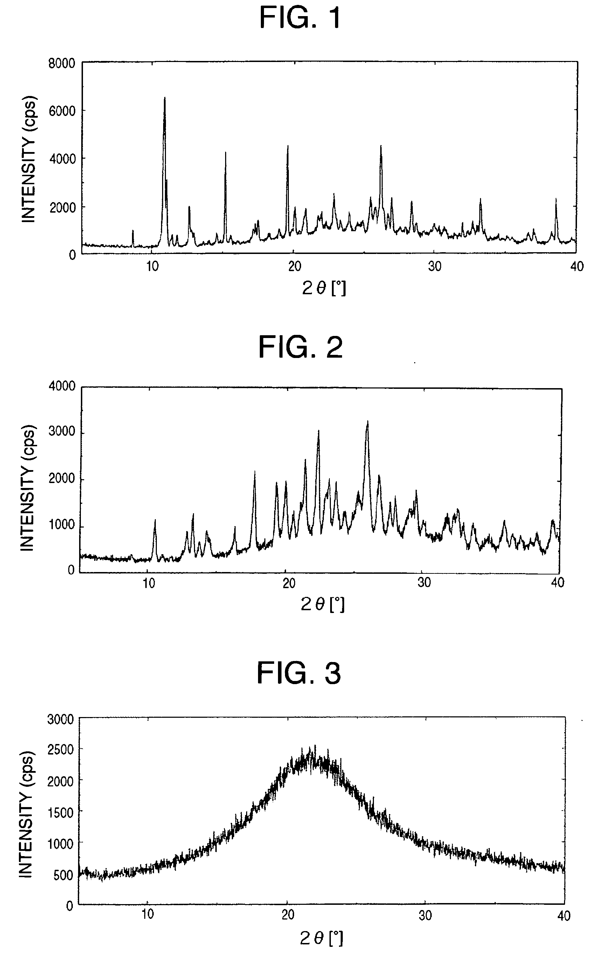

The crystals obtained by the above crystallization method were placed on a sample stage of a powder X-ray diffractometer and analyzed under the conditions shown in Table 1. The powder X-ray diffraction pattern of the resulting crystals is shown in Fig. 1.

**[Table 1]**

| Measurement conditions | |
|---|---|
| Sample holder | Glass |
| Target | Copper |
| Detector | Scintillation counter |
| Tube voltage | 40 KV |
| Tube current | 200 mA |
| Slit | DS 1/2°, RS 0.3mm, SS 1/2° |
| Scanning speed | 5°/min |
| Sampling interval | 0.02° |
| Scanning range | 5 to 40° |
| Goniometer | Vertical goniometer |

### Example 4

### Crystallization of (3E)-1-[(1S)-1-(4-fluorophenyl)ethyl]-3-[3-methoxy-4-(4-methyl-1H-imidazol-1-yl)benzylidene]piperidin-2-one dihydrochloride monohydrate

A suspension of (3E)-1-[(1S)-1-(4-fluorophenyl)ethyl]-3-[3-methoxy-4-(4-methyl-1H-imidazol-1-yl)benzylidene]piperidin-2-one dihydrochloride monohydrate (50 mg) in 2-butanone-1-propanol (5:1,750 µL) was heated to 80°C and the compound was completely dissolved. Then, the solution was gradually cooled overnight. The precipitated solid was separated by filtration and washed with 2-butanone-1-propanol (5:1) to provide 27 mg of a crystalline form.

### Example 5

### Crystallization of (3E)-1-[(1S)-1-(4-fluorophenyl)ethyl]-3-[3-methoxy-4-(4-methyl-1H-imidazol-1-yl)benzylidene]piperidin-2-one dihydrochloride monohydrate

A suspension of (3E)-1-[(1S)-1-(4-fluorophenyl)ethyl]-3-[3-methoxy-4-(4-methyl-1H-imidazol-1-yl)benzylidene]piperidin-2-one dihydrochloride monohydrate (50 mg) in acetone-ethanol (10:1, 500 µl) was refluxed and the compound was completely dissolved. Then, the solution was gradually cooled overnight. The precipitated solid was separated by filtration and washed with acetone-ethanol (10:1) to provide 30 mg of a crystalline form.

### Example 6

### Crystallization of (3E)-1-[(lS)-1-(4-fluorophenyl)ethyl]-3-[3-methoxy-4-(4-methyl-1H-imidazol-1-yl)benzylidenelpiperidin-2-one dihydrochloride monohydrate

A suspension of (3E)-1-[(1S)-1-(4-fluorophenyl)ethyl]-3-[3-methoxy-4-(4-methyl-1H-imidazol-1-yl)benzylidene]piperidin-2-one dihydrochloride monohydrate (50 mg) in hexane-acetonitrile (1:1, 500 µL) was refluxed and the compound was completely dissolved. Then, the solution was gradually cooled overnight. The precipitated solid was separated by filtration and washed with hexane-acetonitrile (1:1) to provide 22 mg of a crystalline form.

### Example 7

### Synthesis of (3E)-1-[(1S)-1-(4-fluorophenyl)ethyl]-3-[3-methoxy-4-(4-methyl-1H-imidazol-1-yl)benzylidene]piperidin-2-one dihydrobromide monohydrate

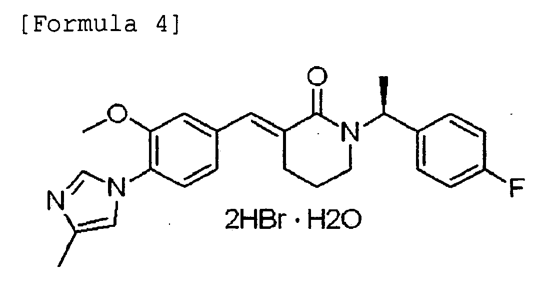

Aqueous hydrogen bromide (48%, 16.2 µL) dissolved in ethyl acetate (1 mL) was added to a solution of (3E)-1-[(1S)-1-(4-fluorophenyl)ethyl]-3-[3-methoxy-4-(4-methyl-1H-imidazol-1-yl)benzylidene]piperidin-2-one (20 mg) in ethyl acetate (1 mL). The mixture was stirred at room temperature. The precipitated solid was separated by filtration and washed with ethyl acetate to provide 8.0 mg of the title compound. The property values of the compound are as follows.
¹ H-NMR (CD₃ OD) δ (ppm) : 1.59 (d, J = 7.2 Hz, 3H), 1.64-1.74 (m, 1H), 1.80-1.87 (m, 1H), 2.43 (d, J = 1.2 Hz, 3H), 2.80-2.84 (m, 2H), 2.99 (ddd, J = 4.0, 6.4, 12.8 Hz, 1H), 3.37 (ddd, J = 4.0, 8.8, 12.8 Hz, 1H), 3.95 (s, 3H), 6.10 (q, J = 7.2 Hz, 1H), 7.07-7.13 (m, 2H), 7.21 (dd, J = 1.2, 8.0 Hz, 1H), 7. 31 (d, J = 1.2 Hz, 1H), 7.36-7.40 (m, 2H), 7.57 (d, J = 8.0 Hz, 1H), 7.60 (t, J = 1.2 Hz, 1H), 7.79-7.81 (m, 1H) 9.16 (d, J = 1.2 Hz, 1H).

### Example 8

### Crystallization of (3E)-1-[(1S)-1-(4-fluorophenyl)ethyl]-3-[3-methoxy-4-(4-methyl-1H-imidazol-1-yl)benzylidene]piperidin-2-one dihydrobromide monohydrate

A suspension of (3E)-1-[(1S)-1-(4-fluorophenyl)ethyl]-3-[3-methoxy-4-(4-methyl-1H-imidazol-1-yl)benzylidene]piperidin-2-one dihydrobromide monohydrate (100 mg) in ethyl acetate-ethanol (4.4:1.0, 2.45 mL) was refluxed and the compound was completely dissolved. Then, the solution was left to stand at room temperature overnight. The precipitated solid was separated by filtration and washed with ethyl acetate-ethanol (4.4:1) to provide 35 mg of a crystalline form.

### Example 9

### Crystallization of (3E)-1-[(lS)-1-(4-fluorophenyl)ethyl]-3-[3-methoxy-4-(4-methyl-1H-imidazol-1-yl)benzylidene]piperidin-2-one dihydrobromide monohydrate

A suspension of (3E)-1-[(1S)-1-(4-fluorophenyl)ethyl]-3-[3-methoxy-4-(4-methyl-1H-imidazol-1-yl)benzylidene]piperidin-2-one dihydrobromide monohydrate (100 mg) in acetonitrile (2 mL) was heated to 80°C and the compound was completely dissolved. Then, the solution was gradually cooled overnight. The precipitated solid was separated by filtration and washed with acetonitrile to provide 27 mg of a crystalline form.

### Example 10

### Crystallization of (3E)-1-[(1S)-1-(4-fluorophenyl)ethyl]-3-[3-methoxy-4-(4-methyl-1H-imidazol-1-yl)benzylidenelpiperidin-2-one dihydrobromide monohydrate

### (1) Crystallization method

A suspension of (3E)-1-[(1S)-1-(4-fluorophenyl)ethyl]-3-[3-methoxy-4-(4-methyl-1H-imidazol-1-yl)benzylidene]piperidin-2-one dihydrobromide monohydrate (100 mg) in acetonitrile (1.5 mL) was heated to 80°C and the compound was completely dissolved. Then, the solution was gradually cooled overnight. The precipitated solid was separated by filtration and washed with acetonitrile to provide 20 mg of a crystalline form.

### (2) Powder X-ray diffractometry

The crystals of (3E)-1-[(1S)-1-(4-fluorophenyl)ethyl]-3-[3-methoxy-4-(4-methyl-1H-imidazol-1-yl)benzylidenelpiperidin-2-one dihydrobromide monohydrate obtained by the above crystallization method were placed on a sample stage of a powder X-ray diffractometer and analyzed under the measurement conditions described in Table 1 of Example 3. The powder X-ray diffraction pattern of the resulting crystals is shown in Fig. 2.

### Example 11

### Synthesis of (3E)-1-[(1S)-1-(4-fluorophenyl)ethyl]-3-[3-methoxy-4-(4-methyl-1H-imidazol-1-yl)benzylidene]piperidin-2-one monofumarate

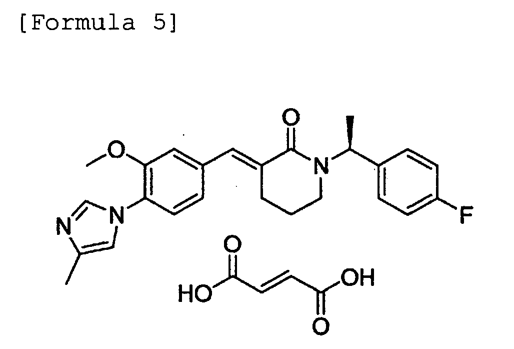

Fumaric acid (13.8 mg) dissolved in THF-diethyl ether (1:1; 2 mL) was added to a solution of (3E)-1-[(1S)-1-(4-fluorophenyl)ethyl]-3-[3-methoxy-4-(4-methyl-1H-imidazol-1-yl)benzylidene]piperidin-2-one (50 mg) in THF-diethyl ether (1:1; 2 mL) at room temperature. The reaction solution was concentrated under reduced pressure to provide 64 mg of the title compound. The property values of the compound are as follows.
¹H-NMR (CD₃ OD) δ (ppm) : 1.58 (d, J = 7.2 Hz, 3H), 1.63-1.90 (m, 2H), 2.30 (s, 3H), 2.76-2.90 (m, 2H), 2.94-3.02 (m, 1H), 3.31-3.40 (m, 1H), 3.92 (s, 3H), 6.10 (q, J = 7.2 Hz, 1H), 6.72 (s, 2H), 7.06-7.18 (m, 3H), 7.23-7.28 (m, 2H), 7.34-7.41 (m, 2H), 7.45 (d, J = 8.0 Hz, 1H), 7.79 (s, 1H), 8.28 (s, 1H).

### Example 12

### Synthesis of (3E)-1-[(1S)-1-(4-fluorophenyl)ethyl]-3-[3-methoxy-4-(4-methyl-1H-imidazol-1-yl)benzylidene]piperidin-2-one mono-L-(+)-tartrate

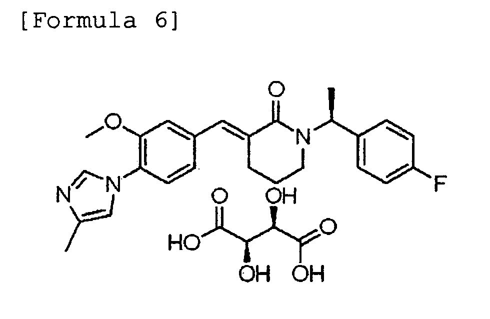

### (1) Synthesis method

L-(+)-tartaric acid (17.9 mg) dissolved in THF-diethyl ether (1:10; 11 mL) was added to a solution of (3E)-1-[(1S)-1-(4-fluorophenyl)ethyl]-3-[3-methoxy-4-(4-methyl-1H-imidazol-1-yl)benzylidene]piperidin-2-one (50 mg) in THF-diethyl ether (1:10; 11 mL) at room temperature. The precipitated solid was separated by filtration and washed with diethyl ether to provide 28 mg of the title compound. The property values of the compound are as follows.
¹ H-NMR (CD₃ OD) δ (ppm) : 1.58 (d, J = 6.4 Hz, 3H), 1.63-1.74 (m, 1H), 1.78-1.89 (m, 1H), 2.30 (s, 3H), 2.79-2.86 (m, 2H), 2.94-3.02 (m, 1H), 3.30-3.40 (m, 1H), 3.92 (s, 3H), 4.48 (s, 2H), 6.10 (q, J = 6.4 Hz, 1H), 7.06-7.18 (m, 3H), 7.22-7.27 (m, 2H), 7.35-7.47 (m, 3H), 7.79 (s, 1H), 8.22 (s, 1H).

### (2) Powder X-ray diffractometry

The amorphous form obtained by the above synthesis method was placed on a sample stage of a powder X-ray diffractometer and analyzed under the measurement conditions described in Table 1 of Example 3. The powder X-ray diffraction pattern of the resulting amorphous form is shown in Fig. 3.

### Example 13

### Synthesis of (3E)-1-[(1S)-1-(4-fluorophenyl)ethyl]-3-[3-methoxy-4-(4-methyl-1H-imidazol-1-yl)benzylidene]piperidin-2-one monomaleate

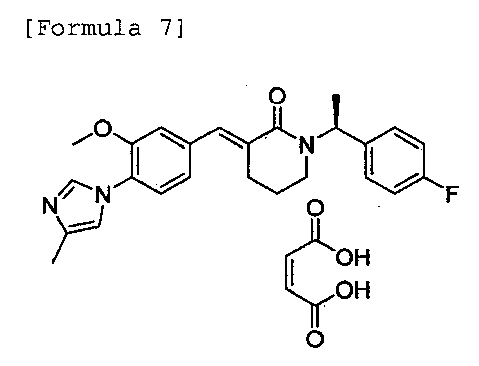

The title compound was synthesized in the same manner as in Example 11.
¹H-NMR (CD₃OD) δ (ppm) : 1.59 (d, J = 6.8 Hz, 3H), 1.65-1.75 (m, 1H), 1.80-1.90 (m, 1H), 2.41 (s, 3H), 2.80-2.86 (m, 2H), 2.95-3.03 (m, 1H), 3.32-3.40 (m, 1H), 3.94 (s, 3H), 6.09 (q, J = 6.8 Hz, 1H), 6.24 (s, 2H), 7.06-7.12 (m, 2H), 7.19 (dd, J = 8.4, 1.6 Hz, 1H), 7.29 (d, J = 1.6 Hz, 1H), 7.35-7.41 (m, 2H), 7.52 (d, J = 1.6 Hz, 1H), 7.35 (d, J = 8.4 Hz, 1H), 7.79 (s, 1H), 8.97 (d, J = 1.6 Hz, 1H) .

### Example 14

### Synthesis of (3E)-1-[(lS)-1-(4-fluorophenyl)ethyl]-3-[3-methoxy-4-(4-methyl-1H-imidazol-1-yl)benzylidene]piperidin-2-one hemisulfate

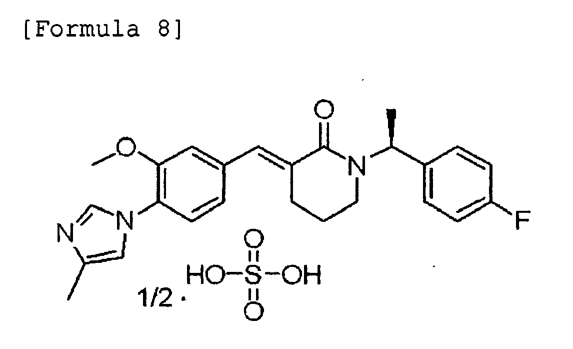

### (1) Synthesis method

Concentrated sulfuric acid (1.25 µL) and water (5 µL) dissolved in ethyl acetate (0.5 mL) were added to a solution of (3E)-1-[(1S)-1-(4-fluorophenyl)ethyl]-3-[3-methoxy-4-(4-methyl-1H-imidazol-1-yl)benzylidene]piperidin-2-one (20 mg) in ethyl acetate (0.5 mL). The mixture was stirred at room temperature. The reaction solution was concentrated under reduced pressure to provide 22.3 mg of the title compound. The property values of the compound are as follows.
¹H-NMR (CD₃ OD) δ (ppm) : 1.60 (d, J = 7.2 Hz, 3H), 1.65-1.75 (m, 1H), 1.81-1.90 (m, 1H), 2.44 (d, J = 1.2 Hz, 3H), 2.81-2.85 (m, 2H), 2.96-3.02 (m, 1H), 3.37 (ddd, J = 12.4, 8.4, 4.0 Hz, 1H), 3.95 (s, 3H), 6.10 (q, J = 7.2 Hz, 1H), 7.07-7.12 (m, 2H), 7.21 (d, J = 8.0 Hz, 1H), 7.30-7.31 (brs, 1H), 7.36-7.40 (m, 2H), 7.56-7.59 (m, 2H), 7.79-7.81 (m, 1H), 9. 15 (brs, 1H).

### (2) Powder X-ray diffractometry

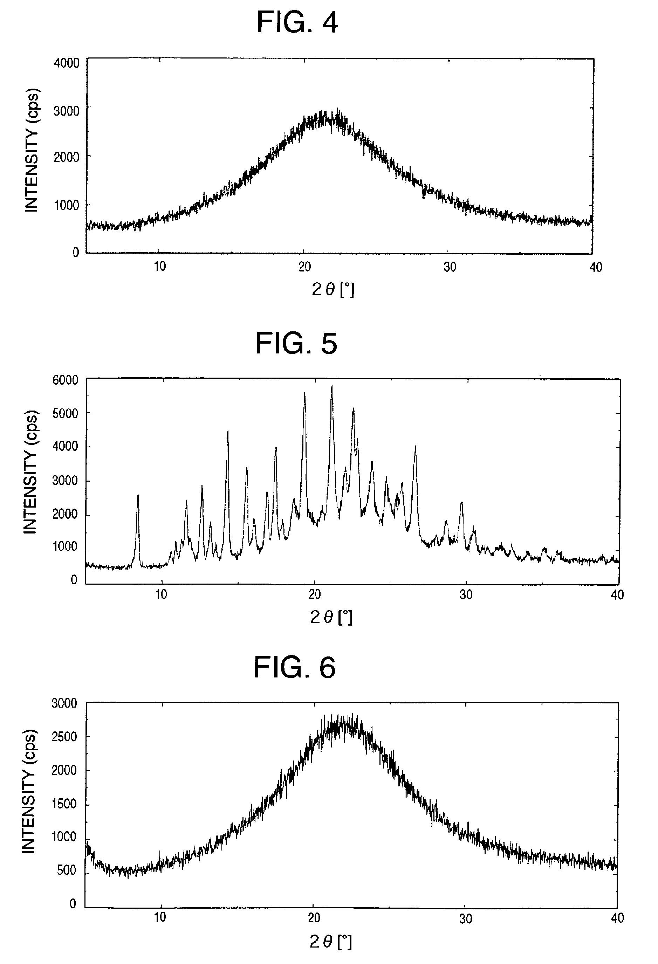

The amorphous form obtained by the above synthesis method was placed on a sample stage of a powder X-ray diffractometer and analyzed under the measurement conditions described in Table 1 of Example 3. The powder X-ray diffraction pattern of the resulting amorphous form is shown in Fig. 4.

### Example 15

### Synthesis of (3E)-1-[(1S)-1-(4-fluorophenyl)ethyl]-3-[3-methoxy-4-(4-methyl-1H-imidazol-1-yl)benzylidene]piperidin-2-one monotosylate

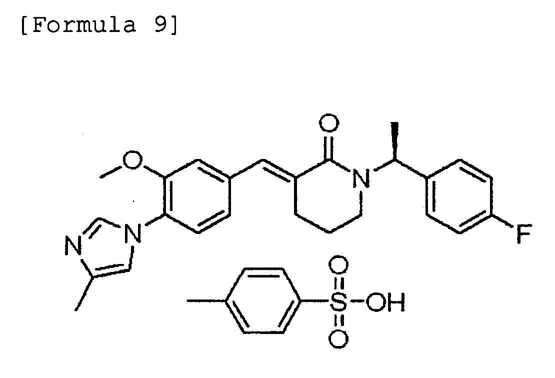

p-Toluenesulfonic acid (8.9 mg) and water (5 µL) dissolved in ethyl acetate (0.5 mL) were added to a solution of (3E)-1-[(1S)-1-(4-fluorophenyl)ethyl]-3-[3-methoxy-4-(4-methyl-1H-imidazol-1-yl)benzylidene]piperidin-2-one (20 mg) in ethyl acetate (0.5 mL). The mixture was stirred at room temperature. The reaction solution was concentrated under reduced pressure to provide 28.9 mg of the title compound. The property values of the compound are as follows.
1 H-NMR (CD₃OD) δ(ppm) : 1.59 (d, J = 7.2 Hz, 3H), 1.66-1.74 (m, 1H), 1.80-1.87 (m, 1H), 2.36 (s, 3H), 2.41 (s, 3H), 2.80-2.84 (m, 2H), 2.96-3.02 (m, 1H), 3.36 (ddd, J = 3.6, 8.4, 12 Hz, 1H), 3.94 (s, 3H), 6.09 (q, J = 7.2 Hz, 1H), 7.07-7.12 (m, 2H), 7.19-7.22 (m, 3H), 7.29 (m, 1H), 7.36-7.39 (m, 2H), 7.54-7.55 (m, 1H), 7.55 (d, J = 8.0 Hz, 1H), 7.68 (d, J = 8.0 Hz, 2H), 7.79-7.81 (m, 1H), 9.04 (d, J = 1.6 Hz, 1H).

### Example 16

### Synthesis of (3E)-1-[(1S)-1-(4-fluorophenyl)ethyl]-3-[3-methoxy-4-(4-methyl-1H-imidazol-1-yl)benzylidene]piperidin-2-one monomesylate

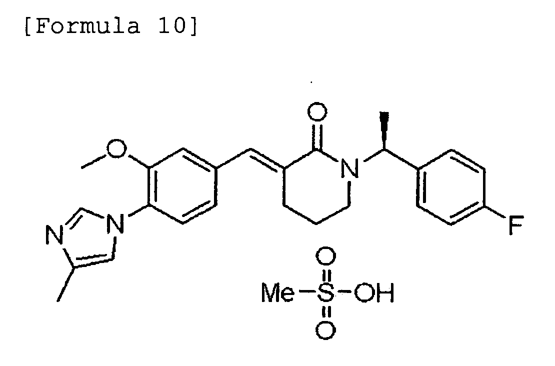

Methane sulfonic acid (3 µL) and water (5 µL) dissolved in ethyl acetate (0.5 mL) were added to a solution of (3E)-1-[(1S)-1-(4-fluorophenyl)ethyl]-3-[3-methoxy-4-(4-methyl-1H-imidazol-1-yl)benzylidene]piperidin-2-one (20 mg) in ethyl acetate (0.5 mL). The mixture was stirred at room temperature. The reaction solution was concentrated under reduced pressure to provide 24.6 mg of the title compound. The property values of the compound are as follows.
¹H-NMR (CD₃OD) δ(ppm): 1.59 (d, J = 7.2 Hz, 3H), 1.65-1.75 (m, 1H), 1.81-1.90 (m, 1H), 2.44 (d, J = 0.8 Hz, 3H), 2.70 (s, 3H), 2.81-2.85 (m, 2H), 2.96-3.02 (m, 1H), 3.37 (ddd, J = 4.0, 8.4, 12.4 Hz, 1H), 3.86 (s, 3H), 6.09 (q, J = 7.2 Hz, 1H), 7.08-7.12 (m, 2H), 7.21 (dd, J = 1.2, 8.4 Hz, 1H), 7.31 (d, J = 0.8 Hz, 1H), 7.36-7.40 (m, 2H), 7.56 (d, J = 8.4 Hz, 1H), 7.59 (t, J = 1.2 Hz, 1H), 7.80 (s, 1H), 9.15 (d, J = 1.6 Hz, 1H).

### Example 17

### Synthesis of (3E)-1-[(1S)-1-(4-fluorophenyl)ethyl]-3-[3-methoxy-4-(4-methyl-1H-imidazol-1-yl)benzylidene]piperidin-2-one phosphate 1-propanolate

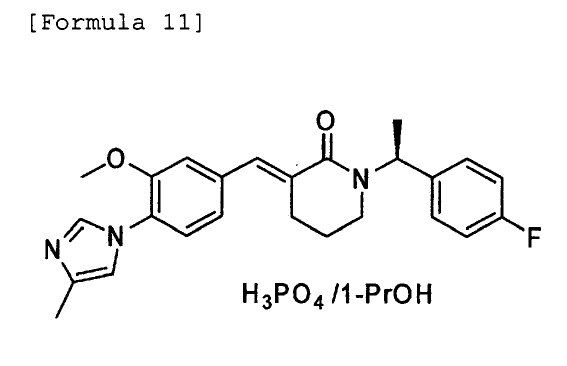

### (1) Synthesis method

Phosphoric acid (13.9 µL, 0.238 mmol) was added to a solution of (3E)-1-[(1S)-1-(4-fluorophenyl)ethyl]-3-[3-methoxy-4-(4-methyl-1H-imidazol-1-yl)benzylidenelpiperidin-2-one (100 mg, 0.248 mmol) in 1-propanol (1.0 mL) at room temperature. After confirming precipitation of the crystals, 1-propanol (1.0 mL) was further added and the mixture was stirred for 15 minutes. Thereafter, the crystals were collected by filtration. The crystals were washed with 1-propanol and then dried under reduced pressure to provide the title compound as white crystals (113.3 mg, 0.196 mmol, 82.3% yield).
¹H-NMR (400M Hz, DMSO-d6) δ(ppm) : 0.82 (3H, t, J = 7 Hz), 1.40 (2H, tq, J = 7, 7 Hz), 1.49 (3H, d, J = 7 Hz), 1.55-1.66 (1H, m), 1.71-1.81 (1H, m), 2.15 (3H, s), 2.73-2.80 (2H, m), 2.81 (1H, ddd, J = 12, 6, 4 Hz), 3.29 (1H, ddd, J = 12, 9, 4 Hz), 3.32 (2H, t, J = 7 Hz), 3.84 (3H, s), 5.97 (1H, q, J = 7 Hz), 7.11 (1H, dd, J = 8, 2 Hz), 7.15-7.20 (3H, m), 7.26 (1H, d, J = 2 Hz), 7.32-7.36 (2H, m), 7.39 (1H, d, J = 8 Hz), 7.70 (1H, s), 7.84 (1H, d, J = 1 Hz)

### (2) Powder X-ray diffractometry

The crystals obtained by the above synthesis method were placed on a sample stage of a powder X-ray diffractometer and analyzed under the measurement conditions described in Table 1 of Example 3. The powder X-ray diffraction pattern of the resulting crystals is shown in Fig. 5.

### Example 18

### Synthesis of (3E)-1-[(1S)-1-(4-fluorophenyl)ethyl]-3-[3-methoxy-4-(4-methyl-1H-imidazol-1-yl)benzylidene]piperidin-2-one tetrafluoroborate

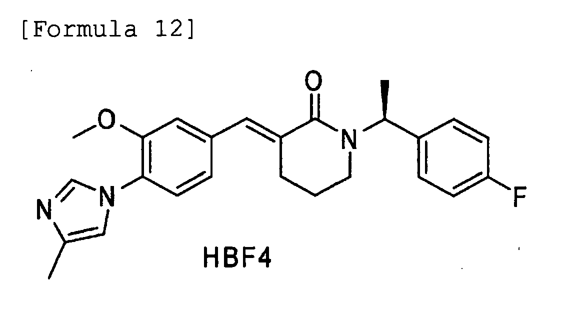

Tetrafluoroboric acid (20.9 µL, 0.238 mmol) was added to a solution of (3E)-1-[(1S)-1-(4-fluorophenyl)ethyl]-3-[3-methoxy-4-(4-methyl-1H-imidazol-1-yl)benzylidene]piperidin-2-one (100 mg, 0.248 mmol) in ethyl acetate (2.5 mL) at room temperature. Then, the mixture was cooled in an ice bath. After stirring for 15 minutes, the crystals were collected by filtration and dried under reduced pressure to provide the title compound as white crystals (55.7 mg, 0.110 mmol, 46.1% yield).
¹H-NMR (400M Hz, DMSO-d6) δ(ppm) : 1.50 (3H, d, J = 7 Hz), 1.56-1.66 (1H, m), 1.72-1.82 (1H, m), 2.33 (3H, d, J = 1 Hz), 2.75-2.78 (2H, m), 2.89 (1H, ddd, J = 12, 6, 4 Hz), 3.30 (1H, ddd, J = 12, 9, 4 Hz), 3.88 (3H, s), 5.97 (1H, q, J = 7 Hz), 7.14-7.22 (3H, m), 7.33-7.38 (3H, m), 7.59 (1H, d, J = 8 Hz), 7.71 (1H, t, J = 1 Hz), 7.73 (1H, s), 9.27 (1H, d, J = 2 Hz)

### Example 19

### Synthesis of amorphous form of (3E)-1-[(lS)-1-(4-fluorophenyl)ethyl]-3-[3-methoxy-4-(4-methyl-1H-imidazol-1-yl)benzylidene]piperidin-2-one phosphate

### (1) Synthesis method

Phosphoric acid (13.9 µL, 0.238 mmol) was added to a solution of (3E)-1-[(1S)-1-(4-fluorophenyl)ethyl]-3-[3-methoxy-4-(4-methyl-1H-imidazol-1-yl)benzylidenelpiperidin-2-one (100 mg, 0.248 mmol) in ethyl acetate (2.0 mL) at room temperature. The mixture was stirred for two hours. After confirming precipitation of the solid, the mixture was cooled in an ice bath and stirred for five minutes. The solid was collected by filtration, washed with ethyl acetate and then dried under reduced pressure to provide the title compound as a white solid (63.3 mg, 0.122 mmol, 51.3% yield).
¹H-NMR (400M Hz, DMSO-d6) δ(ppm) : 1.49 (3H, d, J = 7 Hz), 1.55-1.65 (1H, m), 1.70-1.81 (1H, m), 2.16 (3H, s), 2.72-2.81 (2H, m), 2.87 (1H, ddd, J = 4, 6, 12 Hz), 3.29 (1H, ddd, J = 12, 9, 4 Hz), 3.85 (3H, s), 5.97 (1H, q, J = 7 Hz), 7.10-7.20 (4H, m), 7.27 (1H, d, J = 1 Hz), 7.32-7.36 (2H, m), 7.41 (1H, d, J = 8 Hz), 7.70 (1H, s), 7 . 92 (1H, d, J = 1 Hz)

### (2) Powder X-ray diffractometry

The amorphous form obtained by the above synthesis method was placed on a sample stage of a powder X-ray diffractometer and analyzed under the measurement conditions described in Table 1 of Example 3. The powder X-ray diffraction pattern of the resulting amorphous form is shown in Fig. 6.

### Example 20

### Synthesis of (3E)-1-[(1S)-1-(4-fluorophenyl)ethyl]-3-[3-methoxy-4-(4-methyl-1H-imidazol-1-yl)benzylidene]piperidin-2-one perchlorate

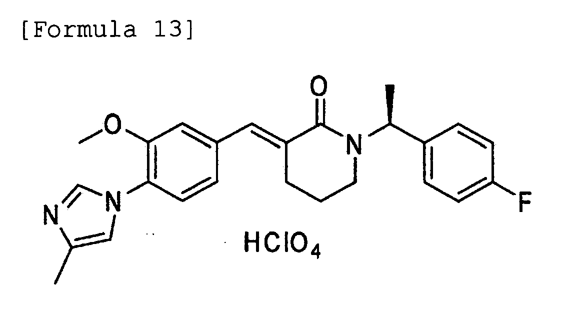

Perchloric acid (60% in water, 39.9 µL, 0.238 mmol) was added to a solution of (3E)-1-[(1S)-1-(4-fluorophenyl)ethyl]-3-[3-methoxy-4-(4-methyl-1H-imidazol-1-yl)benzylidene]piperidin-2-one (100 mg, 0.248 mmol) in ethyl acetate (2.0 mL) at room temperature. Then, the mixture was stirred in an ice bath for five minutes. The crystals were collected by filtration, washed with ethyl acetate and then dried under reduced pressure to provide the title compound as a white solid (121.9 mg, 0.234 mmol, 98.3% yield).
¹H-NMR (400M Hz, DMSO-d6) δ(ppm) : 1.50 (3H, d, J = 7 Hz), 1.56-1.65 (1H, m), 1.72-1.82 (1H, m), 2.34 (3H, s), 2.75-2.78 (2H, m), 2.86-2.92 (1H, m), 3.30 (1H, ddd, J = 12, 9, 4 Hz), 3.88 (3H, s), 5.97 (1H, q, J = 7 Hz), 7.16-7.22 (3H, m), 7.32-7.38 (3H, m), 7.60 (1H, d, J = 8 Hz), 7.73 (2H, s), 9.34 (1H, s)

### INDUSTRIAL APPLICABILITY

The present invention can provide crystals of various salts of the compound (1) or solvates thereof which can be used as drug substances, including (3E)-1-[(1S)-1-(4-fluorophenyl)ethyl]-3-[3-methoxy-4-(4-methyl-1H-imidazol-1-yl)benzylidene]piperidin-2-one dihydrochloride monohydrate and (3E)-1-[(1S)-1-(4-fluorophenyl)ethyl]-3-[3-methoxy-4-(4-methyl-1H-imidazol-1-yl)benzylidene]piperidin-2-one dihydrobromide monohydrate. The present invention can also provide amorphous forms of various salts of the compound (1) or solvates thereof, including (3E)-1-[(1S)-1-(4-fluorophenyl)ethyl]-3-[3-methoxy-4-(4-methyl-1H-imidazol-1-yl)benzylidene]piperidin-2-one monotartrate and (3E)-1-[(1S)-1-(4-fluorophenyl)ethyl]-3-[3-methoxy-4-(4-methyl-1H-imidazol-1-yl)benzylidene]piperidin-2-one hemisulfate. These are crystal polymorphs or amorphous forms of salts of (3E)-1-[(1S)-1-(4-fluorophenyl)ethyl]-3-[3-methoxy-4-(4-methyl-1H-imidazol-1-yl)benzylidene]piperidin-2-one or solvates thereof which are suitable for pharmaceutical preparations.

### BRIEF DESCRIPTION OF THE DRAWINGS

[Fig. 1] Fig. 1 is the powder X-ray diffraction pattern of a crystal of compound (1) dihydrochloride monohydrate. The horizontal axis shows a diffraction angle (2θ), and the vertical line shows a peak intensity.
[Fig. 2] Fig. 2 is the powder X-ray diffraction pattern of a crystal of compound (1) dihydrobromide monohydrate. The horizontal axis shows a diffraction angle (2θ), and the vertical line shows a peak intensity.
[Fig. 3] Fig. 3 is the powder X-ray diffraction pattern of an amorphous form of compound (1) monotartrate. The horizontal axis shows a diffraction angle (2θ), and the vertical line shows a peak intensity.
[Fig. 4] Fig. 4 is the powder X-ray diffraction pattern of an amorphous form of compound (1) hemisulfate. The horizontal axis shows a diffraction angle (2θ), and the vertical line shows a peak intensity.
[Fig. 5] Fig. 5 is the powder X-ray diffraction pattern of compound (1) phosphate 1-propanolate. The horizontal axis shows a diffraction angle (2θ), and the vertical line shows a peak intensity.
[Fig. 6] Fig. 6 is the powder X-ray diffraction pattern of an amorphous form of compound (1) phosphate. The horizontal axis shows a diffraction angle (2θ), and the vertical line shows a peak intensity.

## Claims

1. A salt comprising one acid selected from the group consisting of an inorganic acid, an organic carboxylic acid and an organic sulfonic acid and (3E)-1-[(1S)-1-(4-fluorophenyl)ethyl]-3-[3-methoxy-4-(4-methyl-1H-imidazol-1-yl)benzylidene]piperidin-2-one, or a solvate thereof.

2. The salt or the solvate thereof according to claim 1, wherein the acid is an organic carboxylic acid.

3. The salt or the solvate thereof according to claim 1 or 2, wherein the organic carboxylic acid is acetic acid, oxalic acid, maleic acid, tartaric acid, malonic acid, fumaric acid or citric acid.

4. The salt or the solvate thereof according to claim 1, wherein the acid is an organic sulfonic acid.

5. The salt or the solvate thereof according to claim 1 or 4, wherein the organic sulfonic acid is methanesulfonic acid, trifluoromethanesulfonic acid, ethanesulfonic acid, benzenesulfonic acid, toluenesulfonic acid or camphorsulfonic acid.

6. The salt or the solvate thereof according to claim 1, wherein the acid is an inorganic acid.

7. The salt or the solvate thereof according to claim 1 or 6, wherein the inorganic acid is tetrafluoroboric acid, hydrofluoric acid, hydrochloric acid, hydrobromic acid, hydriodic acid, sulfuric acid, nitric acid, perchloric acid, phosphoric acid, carbonic acid or bicarbonic acid.

8. The salt or the solvate thereof according to claim 1, 6 or 7, wherein the inorganic acid is tetrafluoroboric acid, hydrofluoric acid, hydrochloric acid, hydrobromic acid or hydriodic acid.

9. The salt or the solvate thereof according to claim 1, 6, 7 or 8, wherein the inorganic acid is hydrochloric acid.

10. The salt or the solvate thereof according to claim 1, 6, 7 or 8, wherein the inorganic acid is hydrobromic acid.

11. The solvate according to any one of claims 1 to 10, wherein the solvate is a hydrate or an alcoholate.

12. The solvate according to claim 11, wherein the alcoholate is a 1-propanolate.

13. The salt or the solvate thereof according to any one of claims 1 to 12, which is a crystalline form or an amorphous form.

14. A crystal of (3E)-1-[(1S)-1-(4-fluorophenyl)ethyl]-3-[3-methoxy-4-(4-methyl-1H-imidazol-1-yl)benzylidene]piperidin-2-one dihydrochloride monohydrate having a diffraction peak at a diffraction angle (2θ ± 0.2°) of 10.9° in powder X-ray diffractometry.

15. The crystal according to claim 14, further having a diffraction peak at a diffraction angle (2θ ± 0.2°) of 11.1°.

16. The crystal according to claim 14 or 15, further having a diffraction peak at a diffraction angle (2θ ± 0.2°) of 12.7°.

17. The crystal according to any one of claims 14 to 16, further having a diffraction peak at a diffraction angle (2θ ± 0.2°) of 13.0°.

18. The crystal according to any one of claims 14 to 17, further having a diffraction peak at a diffraction angle (2θ ± 0.2°) of 17.2°.

19. The crystal according to any one of claims 14 to 18, further having a diffraction peak at a diffraction angle (2θ ± 0.2°) of 20.7°.

20. The crystal according to any one of claims 14 to 19, further having a diffraction peak at a diffraction angle (2θ ± 0.2°) of 24.5°.

21. The crystal according to any one of claims 14 to 20, further having a diffraction peak at a diffraction angle (2θ ± 0.2°) of 25.6°.

22. A crystal of (3E)-1-[(1S)-1-(4-fluorophenyl)ethyl]-3-[3-methoxy-4-(4-methyl-1H-imidazol-1-yl)benzylidene]piperidin-2-one dihydrobromide monohydrate having a diffraction peak at a diffraction angle (2θ ± 0.2°) of 10.5° in powder X-ray diffractometry.

23. The crystal according to claim 22, further having a diffraction peak at a diffraction angle (2θ ± 0.2°) of 13.2°.

24. The crystal according to claim 22 or 23, further having a diffraction peak at a diffraction angle (2θ ± 0.2°) of 17.7°.

25. The crystal according to claim 23 or 24, further having a diffraction peak at a diffraction angle (2θ ± 0.2°) of 21.4°.

26. The crystal according to any one of claims 23 to 25, further having a diffraction peak at a diffraction angle (2θ ± 0.2°) of 22.3°.

27. The crystal according to any one of claims 23 to 26, further having a diffraction peak at a diffraction angle (2θ ± 0.2°) of 39.4°.

28. A crystal of (3E)-1-[(1S)-1-(4-fluorophenyl)ethyl]-3-[3-methoxy-4-(4-methyl-1H-imidazol-1-yl)benzylidene]piperidin-2-one phosphate 1-propanolate having a diffraction peak at a diffraction angle (2θ ± 0.2°) of 8.4°.

29. The crystal according to claim 28, further having a diffraction peak at a diffraction angle (2θ ± 0.2°) of 14.3°.

30. The crystal according to claim 28 or 29, further having a diffraction peak at a diffraction angle (2θ ± 0.2°) of 19.3°.

31. The crystal according to any one of claims 28 to 30, further having a diffraction peak at a diffraction angle (2θ ± 0.2°) of 21.1°.

32. The crystal according to any one of claims 28 to 30, further having a diffraction peak at a diffraction angle (2θ ± 0.2°) of 29.6°.

33. (3E)-1-[(1S)-1-(4-fluorophenyl)ethyl]-3-[3-methoxy-4-(4-methyl-1H-imidazol-1-yl)benzylidene]piperidin-2-one phosphate which is an amorphous form.

34. (3E)-1-[(1S)-1-(4-fluorophenyl)ethyl]-3-[3-methoxy-4-(4-methyl-1H-imidazol-1-yl)benzylidene]piperidin-2-one monotartrate which is an amorphous form.

35. (3E)-1-[(1S)-1-(4-fluorophenyl)ethyl]-3-[3-methoxy-4-(4-methyl-1H-imidazol-1-yl)benzylidene]piperidin-2-one hemisulfate which is an amorphous form.

36. The amorphous form according to any one of claims 33 to 35, not comprising a crystalline form.

37. The amorphous form according to any one of claims 33 to 35, not having a diffraction peak in powder X-ray diffractometry.
